(19) Europäisches Patentamt
European Patent Office
Office européen des brevets

(11) EP 0 974 360 A2

(12) **EUROPEAN PATENT APPLICATION**

(43) Date of publication:
**26.01.2000 Bulletin 2000/04**

(51) Int Cl.[7]: **A61K 38/40**, A61K 38/17

(21) Application number: **98203765.7**

(22) Date of filing: **06.11.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **26.06.1998 NL 1009505**
**09.10.1998 NL 1010284**

(71) Applicant: **N.V. Nutricia**
**NL-2712 HM Zoetermeer (NL)**

(72) Inventors:
- **Swart, Pieter Jacob**
  **9712 KD Groningen (NL)**
- **Kuipers, Maria Elizabeth**
  **9712 KD Groningen (NL)**
- **Meijer, Dirk Klaas Fokke**
  **9724 AN Groningen (NL)**

(74) Representative: **Duxbury, Stephen**
**Arnold & Siedsma,**
**Sweelinckplein 1**
**2517 GK Den Haag (NL)**

Remarks:
The applicant has subsequently filed a sequence listing and declared, that it includes no new matter.

(54) **Pharmaceutical preparations for use in combatting or preventing surface infections caused by microorganisms**

(57) The invention relates to a medicament for treatment and/or preventment of infections caused by bacteria, fungi, viri and the like, inflammations and/or tumors, said medicament comprising an active amount of a polycationic peptide or protein, and a buffer for maintaining the pH of treatable tissue within a preselected range.

EP 0 974 360 A2

## Description

The invention relates to pharmaceutical preparations for use in combatting, or preventing surface infections caused by microorganisms, especially Candida, and combatting of the side effects thereof.

As is known, bodily infections caused by bacteria, mould and viruses are a large problem. Many of these infections manifest themselves locally at the tissue surface in the form of sores or irritated tissue. In situations wherein the first line of defence of a tissue has been attacked, as is for example the case with open wounds such as occur during operations, with decubitus or burns, or in the case of enteritis, colitis, morbus Crohn, but also in the case of Helicobacter pylori infections, bacterial surface infections, can occur which in turn can lead to a number of negative side effects such as chronic inflammation, sepsis and the like.

Bacterial infections of the mucous membrane in the mouth, such as gingivitis and parodontitis can occur in cases wherein a bad mouth hygiene is present.

Bacterial infections of the skin can manifest themselves as acne vulgaris. Stomatitis and oesophagitis are inflammations which can occur in the mouth and oesophagus as a result of a decreased resistance following chemotherapy of tumours for example.

These type of infections are currently combatted firstly by means of application of antibiotics. Unfortunately, many of the bacteria to be combatted, develop resistance to these antibiotics, whereby the treatment thereof is becoming more and more difficult, and in a number of cases has become impossible.

Virus infections which occur on the surface of tissues often manifest themselves in the form of infections of the herpes virus (aften) or vesicular stomatitis virus.

Mould infections, include for example candidiasis, athlete's foot which can lead to local inflammation and an unpleasant smell.

Since its discovery in 1839 by Langenbeck, the organism initially thought to be the cause of typhus, that of the genus Candida, has been the causative agent of infections in an ever increasing range of anatomical sites and clinical settings. In normal healthy individuals, the yeast Candida is classified as a commensal organism, and can occupy both internal and external surfaces. In normal circumstances an equilibrium between the host and the yeast microflora ensures the avirulent, commensal status of this microorganism. This equilibrium is attained not only by specific immune responses, but also by aspecific factors that are secreted in saliva or mucosal secretions, such as IgA, lysozyme, lactoferrin and histones. This equilibrium can be affected by a range of predispositions, the most common being those patients who are immunocomprimised. For example the onset of candidiasis in people infected with the human immunodeficiency virus (HIV) has been so closely related to manifestation of AIDS and AIDS related diseases, that this is one factor that is used by the Center for Disease Control to define AIDS. Although C. albicans has been implicated in the early stages of AIDS, infections due to C. glabrata and C. krusei are becoming more widespread in late stage AIDS.

Candidiasis, including for example moniliasis, i.e. as outlined above, an infection caused by one of the Candida fungus types, for example Candida albicans, Candida tropicana or Candida glabrata, can occur as the defense system of a living being, human or animal, deteriates whereby Candidas present in the body can no longer be kept under control. This type of situation occurs for example with cancer patients who have been subjected to chemotherapy or radiation therapy, but also with AIDS patients during the later phases of sickness, patients having used steroids during an extensive period of time, such as transplantation patients, also transplantation patients using medicaments in order to depress the immune reaction, certain groups of operation patients, diabetes mellitis patients, sick babies suffering from sprue, which can occur quite often after an antibiotic treatment, and certain groups of women. The infection can manifest itself and the surface of the complete gastrointestinal tract or in the urogenital area, for example from the mouth to the bronchi, oesophagus and gut but also the vagina/vulva can be infected by this sickness.

Before the emergence of the HIV epidemic, oral mycotic infections were treated with the polyene antifungals such as the amphotericin B or nystatin, and with azoles such as miconazole or clotrimazole. The high relapse rate in HIV-positive patients and reported toxic side effects of amphotericin B has led to the use of azoles as the first line treatment. Because ketoconazole and itraconazole are not as readily absorbed, their use has been limited. And although fluconazole has become the agent of choice, its wide spread use has resulted in an increase in resistance of Candida to its antifungal efficacy. In addition the use of 5-fluorocytosine as antimycotic has led to resistant strains of Candida being clinically significant as well. Because of the rising incidence of failures in the treatment of mycoses in the case of severely immunosuppressed patients, there is need for the development of new therapeutic agents that support the antifungal activity of antimycotica.

Candidiasis is common and can under some circumstances be life threatening. Candidiasis is presently treated with medicines which quite often do not appear to be effective since certain candida progenys have developed a tolerance to the medicines used whereby increasing doses thereof have to be administered in order to control the sickness, or alternatively very toxic medicines have to be used.

Accordingly there is a strong need for easily dosed medicaments which can combat or help prevent candida.

According to a first aspect of the present invention a medicament according to claim 1 is provided.

The inventors have surprisingly shown that the effectivity thereof is pH dependent, and can thus be subjected to a degree of control.

The medicament according to the present inventions can be used for treating tissue infected by microorganisms, especially candida, by enabling the active components of the medicament to come into direct contact with microorganisms during a prolonged period of time. The medicaments can take the form of paste, cream, salve, gel, lotion or spray, according to where the infections occur, i.e. of the skin, foot, rectum, bronchi, anus or vagina. The application form of the medicament can also be applied onto an absorbent, for example a wound dressing.

WO 90/11754 teaches pressurized aerosol formulations for inhalations for example.

WO 94/28911 describes compounds which can alter the pH in the gastrointestinal tract to a desired value.

In cases where, for example, an infection occurs in the digestive tract, especially in the mouth, the medicament can take the form of a sustained released sucking tablet (pastille), capsule or chewing gum. The content of the tablet is preferably locally present for more than 30 minutes and remains active during this time, preferably for longer than 45 minutes and most preferably larger than 90 minutes. If the infections occur in a lower area of the digestive tract, for example the duodenum, the medicament can take the form of a fluid wherein the active components are encapsulated in such a way that they are released at a predetermined position in the digestive tract, for example as microspheres, coated granules or a coated tablet.

A suitable buffer system for the medicament is described in WO 94/28911.

A buffer system for regulating the pH in the mouth between values of 6-6.5 is described in WO 88/02600.

A most suitable buffer system comprises hydroxide, carbonate, or citrate salts of magnesium or zinc.

The medicament preferably comprises a polycationic peptide or protein as defined in claims 3 or 4, and furthermore preferably comprises a buffer, as defined in claim 5, wherein the peptide and buffer are more preferably present in the amounts as defined in claim 6, whereby the pH of treatable tissue is thereby maintained within a preselected range as defined for example in claim 2.

The polycationic peptides and proteins preferably comprise human lactoferrin (h-Lf), bovine lactoferrin (bLf), lactoferricin, conalbumin, ovotransferrin, the polycationic peptides which occur in these proteins, as discussed in EP 0 503 939, 0 474 506, 0 510 912 or 0 438 750, hydrolysates of these proteins which comprise the polycationic peptides, the chemical derivatives of these proteins such as the aconytyl or succinilated derivatives, wherein the positive groups are protected, as described in EP 0 406 416 and 0 575 432 and indolicidin analogs. Also comprised are polycations of the family of alfa or beta defensins, preferably defensins isolatable from neutrophyles or from Paneth cells (see for example EP 0 689 550 and EP 0 750 506), such as the magainins such as type 1 or type 2, which for example can be isolated from mucous from the epitherial tissue of tongues or frogs (see WO 95-32287), human defensins such as HNP-1 or HNP-3, rat defensins, such as NP-1 or NP-2, or the cecropins type A or B, the protegrins, from leukocytes (see WO 97-18826), the polycations isolatable from insects (see WO 97-30082), and the histones as defined in the Merck Index, 10'th edition, page 683. Lactoferrin is preferably not denatured.

Other suitable polycationic peptides include the hydrolysates of lactoferrin and cation rich peptides originating from lactoferrin.

If lactoferrin is used, both the 100% iron saturation form and the apo-form can be used as well as mixtures thereof. The amount of lactoferrin in the product should ensure that the tissue to be treated is exposed to a concentration of at least 0.1 mg/ml and preferably more than 1 mg/ml for microorganisms sensitive to the current medicines, and more than 10 mg/ml and preferably more than 40 mg/ml for insensitive microorganisms. Dependent on the application form, at least 0.5 uMol (0.4 g) lactoferrin and preferably more than 5 uMol lactoferrin should be present. The concentration in a salve, cream, lotion or spray is preferably at least 2.5 nMol/ml product and preferably more than 0.12 uMol (0.1 g) lactoferrin per ml product.

The amount of buffer should be high enough in order to keep the pH of the mucous layer of the tissue between a pH value of most preferably between 7 and 8 and maintain this pH for at least 30 and preferably 45 minutes during which time the active component is in contact with the tissue. In order to achieve this, the pH of the medicament can be 7.2, preferably above pH 7.4. When having the form of a tablet or gum (1.2 g), at least 1 mMol of the buffer should be present, preferably more than 2 mMol and in the case of a salve, gel or lotion, preferably more than 10 uMol is present. It is important that the pH of the tissue does not rise above 8.0 since this has an irritating effect and affects the working of the tissue. It can occur that the pH of the medicament lies a little above 8.0, however this is determined to quickly enable a pH value of between 7 and 8 to ensue. The pH value of spittle, during the residence of the medicament tablet in the mouth, should preferably have a pH of above 8 for no longer than 2 minutes and most preferably assume a pH of between 7 and 8 during 60 minutes.

Examples of buffers are detailed in Table 1 below. If needed, more concentrated buffers can be comprised in the medicament.

Table 1:

| buffer systems in water, pH measured at 18°C. | | | | |
|---|---|---|---|---|
| | pH 7.4 | 7.6 | 7.8 | 8.0 |
| a/borax (0.05 M) | 11 ml | 15 | 21 | 27 |
| boric acid (0.2 M) | 89 ml | 85 | 79 | 73 |
| b/borax (0.05 M) | | 52 | 54 | 56 |
| 0.1 N HCl | | 48 | 46 | 44 |
| c/Na2HPO4 (66.7 mM) | 8 | | 9 | |
| KH2PO4 (39.78 mM) | 2 | | 1 | |
| d/$Na_2HPO_4$ (0.2 M) | 90.85 | 93.65 | 95.75 | 97.25 |
| Citric Acid (0.1 M) | 9.15 | 6.35 | 4.25 | 2.75 |

94.7 ml 0.2 M $Na_2$HPO4 mixed with 4.3 ml 0.1 M citric acid provides a buffer system having a pH of 7.7. Other examples of buffer systems include the so-called biological buffers. Compounds such as POPSO (piperazine-N,N'-bis [2-hydroxypropanesulfonic acid]), TAPSO (3- [N-tris (hydroxymethyl)methylamino]-2-hydroxypropanesulfonic acid) or HEPES (N- [2-hydroxyethyl]piperazine-N'-[2-ethanesulfonic acid]) can also be used. A solution of 0.4 M in water is titrated with 0.2 N sodiumhydroxide solution until the desired pH is achieved. After drying thereof, a powder is provided which can be used in tablets.

Buffer systems can also be used such as described in WO 94-28911 for enteral applications or in EP 0 381 414 for oral applications. Preferably calcium phosphate is not added due to the bad solubility thereof and unpleasant taste.

In order to maintain a pH for longer than about 30 minutes in the mouth at a value of between 7 and 8, an acid neutralizing agent is preferably added in an amount of for example between 0.5-100 and preferably 0.8-20 milliequivalents per medicament form.

The medicament may also comprise other active ingredients, see for example claim 7, which ingredients preferably provide no acidifying effect.

The medicament can also furthermore comprise standard known agents, as described in claim 8.

The inventors have shown that a combination of the polycationic or protein as detailed above with these standard agents provides an unexpected synergistic effect.

Lysozymes can also be comprised in the medicament, and as is known, in this case an amount of bicarbonate (HCO3-) and thiocyanate (SCN-) should also be present. The concentration of lysozyme will in general be between 10 and 1000 mg per tablet and 5-100 mg/ml in the case of the creams, gels, lotions and sprays.
Immunoglobulins such as specific sIgA, IgM or IgG against pathogens may also be included.

A very good activity against microorganisms can be obtained when surface active compounds, such as alkylene glycolmonoethyl ethers or monoalkylglyceride esters are also comprised in the medicament.

Compounds are also preferably comprised in the medicament which aid in tissue repair such as metal ions for example zinc. Compounds which may be taken up into the medicament are glutamine, 0.1-0.6 g/g tablet, nucleotides, 1-100 ug/ml cream or 0.1-10 mg/tablet, or growth factors such as for example platelet-derived growth factor or epidermal growth factor in an amount of 10-1000 ug/ml cream or 0.1-50 mg/g tablet.

Furthermore the medicament may comprise vitamins and minerals if required by specific patient groups. For example, these may be present at 1-4 x the daily recommended amount, especially for the vitamins B12, A, B6 and folic acid or metabolic equivalents thereof.

According to a second aspect of the present invention there is provided a medicament according to claim 11.

As detailed above, the inventors have shown that such a medicament provides an unexpected synergistic working.

The invention will now be further illustrated by way of the following examples and results, with reference to the tables and figures, wherein the figures show:

Figure 1: the pH effect on the growth of C. glabrata yl10 in SLM;
Figure 2: the pH effect on the growth of C. glabrata Y110 in RPMI;
Figure 3 (a-i) : the influence of pH and medium on the candida growth inhibition by lactoferrin in SLM-Y110;
Figure 4 (a-i) : the influence of pH and medium on the candida growth inhibition by lactoferrin in RPMI-Y110;
Figure 5 (a-i) : the influence of pH and medium on the candida growth inhibition by fluconazole in SLM-Y110;
Figure 6 (a-i) : the influence of pH and medium on the candida growth inhibition by fluconazole in RPMI) Y110;
Figure 7 (a-h) : the influence of pH and medium on the candida growth inhibition by lactoferrin in SLM-Y 127;

Figure 8: combined inhibitory effects of lactoferrin and fluconazole on the growth of Candida glabrata isolate Y110. Presented is the top elevation of a three-dimensional dose response graph. The amount of

Figure 9: combined inhibitory effects of lactoferrin and fluconazole on the growth of C. glabrata isolate Y110. The graph demonstrates the amount of synergy (i.e. potentiation of inhibition above expected additivity) observed with the combination of the two compounds. Presented is the front elevation of the synergy plot. The amount of synergy is indicated by the grey coloured bar at the right.

Figure 10: combined inhibitory effects of lactoferrin and fluconazole on the growth of Candida albicans isolate Y127. Presented is the top elevation of a three-dimensional dose response graph. The amount of inhibition of the Candida growth is indicated by the right positioned grey colour bar.

Figure 11: combined inhibitory effects of lactoferrin and fluconazole on the growth of C. albicans isolate Y127. The graph demonstrates the amount of synergy (i.e. potentiation of inhibition above expected additivity) observed with the combination of the two compounds. Presented is the front elevation of the synergy plot. The amount of synergy is indicated by the grey coloured bar at the right.

Figure 12: combined inhibitory effects of lactoferrin and amphotericin B on the growth of Candida glabrata isolate yl10. Presented is the top elevation of a three-dimensional dose response graph. The amount of inhibition of the Candida growth is indicated by the right positioned grey colour bar.

Figure 13: combined inhibitory effects of lactoferrin and amphotericin-B on the growth of Candida glabrata isolate yl10. The graph demonstrates the amount of synergy (i.e. potentiation of inhibition above expected additivity) observed with the combination of the two compounds.

Presented is the front elevation of the synergy plot. The amount of synergy is indicated by the grey coloured bar at the right.

Figure 14: the extent of growth inhibition of C. glabrata yl10 using lactoferrin and 5-fluorocytosine.

Figure 15: the extent of growth inhibition of C. albicans Y127 using lactoferrin and amphotericin B.

Figure 16: the extent of growth inhibition of C. glabrata Y111 using lactoferrin and fluconazole.

Figure 17: the extent of growth inhibition of C. tropicana Y140 using lactoferrin and 5-fluorocytosine.

Figure 18: synergistic antifungal activity of lactoferrin and amphotericin B.

Figure 19: synergistic antifungal activity of lactoferrin and 5-fluorocytosine.

Figure 20: synergistic antifungal activity of lactoferrin and 5-fluorocytosine.

Figure 21: synergistic antifungal activity of lactoferrin and fluconazole.

Experimental

**1. The influence of the pH on the antifungal activity of lactoferrin against candida species**

Materials and methods

Organisms

Several oral Candida albicans, and C. glabrata isolates, that differ in their susceptibility to antifungal agents, were obtained from the routine microbiology services of the Microbiology Laboratory Academic Hospital Groningen, The Netherlands. All strains were stored on Sabouraud dextrose agar slopes at 4°C. (SDA; Oxiod, Unipath Ltd, U.K.)

Assay media

The antifungal agent free media used, Sabouraud Liquid Media (SLM; Oxoid, Unipath Ltd, UK, pH 5.6) and RPMI 1640 medium (with L-glutamine w/o $NaHCO_3$ supplemented with 2% glucose, pH 7.0, Gibco BRL, Paisley Scotland), were prepared according to manufacturers instructions. When necessary SLM was adjusted to pH 6.5 and 7.5 using NaOH, RPMI was adjusted to pH 5.0 and 6.0 using HCL. The media were sterilized by filtering through 0.2µm filters.

Antifungal agents

Bovine lactoferrin (Numico B.V. Wageningen, The Netherlands) and fluconazole (Diflucan® I.V.; Pfizer B.V., Holland) were dissolved in assay medium in appropriate concentrations. All suspensions were prepared in sterile glass tubes before addition to the microtitre plate.

Inoculum

The yeast isolates were grown on SDA for 24 hours at 35°C in air. Suspensions were made by picking 5 colonies from these cultures. These were suspended in 10 ml SLM, and mixed while incubating for 18 hours at 35°C in air. From this culture, a 1:10 dilution in either SLM or RPMI of the appropriate pH (5.6, 6.5, 7.5 or 5.0, 6.0, 7.0) was incubated and mixed for 5 hours, resulting in a culture in its growth phase. This was vortexed, and the turbidity adjusted to a density of a 0.5 McFarland barium sulfate turbidity standard at 530 nm, resulted in a concentration of $1\times10^6$-$5\times10^6$ cells per ml. From this, the test inoculum was prepared to a concentration of $1\times10^4$ - $5\times10^4$ cells per ml, by a 1:100 dilution in either SLM or RPMI of the appropriate pH. Confirmation of the inoculum size was determined using the Spiral Plater, Model C (Spiral Systems, Inc, Cincinnati, Ohio, USA). 100 µl was automatically plated out onto a plate containing SDA, which was incubated for 18 hours at 35°C in air, and the concentration calculated according to the manufacturers specifications.

Assay format

To a sterile 96-well plastic assay plate, flat bottom with matching covers (Corning Costar, Cambridge, U.K.), 50 µl of test inoculum was added. Appropriate concentrations of the to be tested antifungal agents were added to the wells (75-150 µl). Controls were included for the determination of growth habits of each Candida species without the presence of an antifungal agent. The final volume per well was adjusted to 200 µl with the assay medium used (SLM or RPMI).

Incubation, growth curves and endpoint criteria

After inoculation, plates were incubated for 48 hours at 35°C in air, without agitation. Turbidity measurements were performed at t=0 hours, t=18-24 hours (hourly), and 48 hours, at 630 nm in an automated microplate reader ($El_x$800, Bio-Tek Instruments, Inc, Winooski, VT, USA), after resuspending contents of the wells with a multi-channel pipette. Any bubbles were removed with the tip of a sterile needle. Any wells not producing visually or spectrophotometrically positive growth after 48 hours, were confirmed by inoculating 20 µl of the wells contents onto SDA, which were subsequently incubated for 5 days at 35°C in air. The minimal inhibitory concentration (MIC) was defined as the lowest antifungal agent concentration that substantially inhibited the growth of Candida after 24 hours according to the recommendations by the National Committee for Clinical Laboratory Standards (1995) for the antifungal agents used. All experiments were performed in quadruplicate.

Results

The effect of the pH on the growth of Candida species

When incubating C. glabrata in SLM at three different pH's, it was observed that the growth of this Candida specie was influenced by the pH of the medium. As can be seen in figure 1, the C. glabrata Y110 grew optimally at a pH of 6.5 or 7.5, whereas at a pH of 5.6, normally used in antifungal assays, the growth of the Candida was significantly lower. As a result of this one can expect that the inhibition of the growth of a optimal growing fungus is more difficult to achieve as compared to the inhibition of a fungus that is growing suboptimal. The results of the growth of Candida albicans Y127 in SLM were comparable with the growth curve of Y110 (results not shown). On the other hand, when incubating Y110 in RPMI medium at several pH's, it was observed that growth at pH 5.0 was optimal but that the changes in pH were not so influential on the growth curve of Y110 in RPMI (figure 2) as compared to the results in SLM, wherein AU = absorption units.

The inhibition of the Candida growth at several pH's

Lactoferrin - Y110

A concentration range of lactoferrin was incubated for 24 hours with C. glabrata Y110 in SLM at pH 5.6, 6.5 or 7.5 and differences in inhibition of the Candida growth were observed. As can be seen in figure 3(a-i), at a pH of 5.6, 10 mg/ml lactoferrin was not sufficient for a complete inhibition of the Candida growth, whereas at a pH of 7.5 the pressure of 10 mg/ml lactoferrin resulted in a total blockade of the growth of the Candida specie. This result was similar when tested in RPMI at several pH's (figure 4(a-i)). Again 10 mg/ml lactoferrin was not sufficient for a complete inhibition of the Candida growth at pH 5.0 and 6.0, but at a pH of 7.0 it was able for total inhibition of the growth.

Fluconazole - Y110

The antifungal activity of fluconazole exhibit a similar pattern against Y110 under influence of the pH and medium as compared to the activity of lactoferrin as described above. It can be seen in figure 5(a-i) and figure 6(a-i) that an increase in pH of the medium resulted in a more efficient inhibition of the Candida specie.

Lactoferrin - Y127

In contrast to the results against Y110, the influence of the pH of the SLM on the inhibitory activity of lactoferrin was not so pronounced against Candida Y127, as is seen in figure 7(a-h). However when expressing the antifungal activity of lactoferrin as MIC values (see later) a lower MIC value was detected at a higher pH (Table 1), see below.

The minimal inhibitory concentration (MIC) was defined as the lowest antifungal agent concentration that substantially inhibited the growth of Candida after 24 hours according to the recommendations by the NCCLS for the several antifungal agents used.

The effect of pH and medium on the minimal inhibitory concentration value

The MIC values of lactoferrin and fluconazole against the Candida isolates tested at several pH and media were determined, according to the recommendations by the NCCLS, after 24 hours of incubation of antifungal agent and Candida species. The results are shown in table 2. It can be seen that both fluconazole and lactoferrin are more effective in Candida inhibition at a higher pH. In addition the antifungal effect of fluconazole was not so strikingly influenced by the type of media used. For SLM and RPMI almost similar MIC values were determined. However the type of media is highly influential for the antifungal effect of lactoferrin. A decrease in MIC value of lactoferrin when tested at higher pH, was observed. Furthermore a 30 to 1000 times decrease in MIC value when tested in RPMI medium as compared to SLM, indicated a far more effective inhibition of the Candida growth when tested in RPMI.

Conclusions

Candida isolate incubated in SLM grows at pH 7.5 optimal.

Candida isolate incubated in RPMI grows at pH 5, 6 or 7 almost identical.

The increase in pH of the medium used resulted in a more effective inhibition of the Candida growth by either lactoferrin or fluconazole, a decrease in MIC values was observed.

The antifungal activity of lactoferrin was significantly increased (30 to 1000 times) when tested against a Candida isolate incubated in RPMI at pH 7.5.

Table 2.

| The minimal inhibitory concentrations (MIC) of fluconazole and lactoferrin against Candida isolates incubated at several pH's in SLM or RPMI medium | | | | |
|---|---|---|---|---|
| Candida isolate | Medium | pH | Fluconazole (mg/ml) | Lactoferrin (mg/ml) |
| Y110 | SLM | 5.6 | 0.20 | 28.0 |
| Y110 | SLM | 6.5 | 0.10 | 31.0 |
| Y110 | SLM | 7.5 | 0.10 | 1.27 |
| Y110 | RPMI | 5.0 | 0.25 | >100 |
| Y110 | RPMI | 6.0 | 0.11 | >100 |
| Y110 | RPMI | 7.0 | 0.06 | 0.10 |
| Y127 | SLM | 5.6 | - | 48.9 |
| Y127 | SLM | 6.5 | - | 46.3 |
| Y127 | SLM | 7.5 | - | 24.9 |

**2. Formulations for pastilles (tablets for sucking), were prepared and tested for in vivo degradation and a pH stabilising effect**

| Formulation examples: | | |
|---|---|---|
| (A) Formulation | I | II |

(continued)

| Formulation examples: | | |
|---|---|---|
| Lactoferrin | 8.27 | 7.87 |
| Buffer | 0.6 (6%) | 1.0 (10%) |
| Na-alginate | 1.0 | 1.0 (10%) |
| Aspartam | 0.05 | 0.05 (0.5%) |
| Aerosil | 0.03 | 0.03 (0.3%) |
| Magnesiumstearate | 0.05 | 0.05 (0.5%) |
| Flavoring | 0.05 | |
| Total | 10.0 | 10.0 mg |
| Tablet weights | 302 mg | 321 mg |
| Active compound amount (lactoferrin) is 250 mg per tablet. | | |

Experimental

The buffer system consisted of 189.4 mMol $Na_2HPO_4$ and 4.3 mMol citric acid.

Male and female volunteers placed a tablet in either cheek. The total active compound was 500 mg. During the experiment eating and/or drinking was not allowed. At predetermined times, spittle samples were taken and the pH thereof measured. The remaining material was stored in order to determine the lactoferrin content.

Corresponding to the in vitro degradation, all the volunteers stated that after 2 hours no solid material was left in the mouth.

None of the volunteers stated any discomfort.

Results

In the case that the initial pH was lower than 6.5, an increase in spittle pH was found for both formulation I and II. The mean end pH was found to be between 7 and 8, in which range, on the basis of the in vitro experiments, a maximum anti-Candida effect of lactoferrin can be expected.

| B. Anti-Candida tablet | | |
|---|---|---|
| Ingredients | Amount per tablet (ca. 1.2 g) | Per batch of 400 kg |
| Colostrum WPI extract | 600 mg | 172 kg |
| Dextrose (Emdex) | 280 | 95 |
| Sodium bicarbonate | 100 | 34 |
| Potassium bicarbonate | 100 | 34 |
| Sorbitol | 56 | 19 |
| Magnesium bicarbonate | 24 | 9.1 |
| Peppermint | 38 | 13 |
| Dibasicmagnesiumphosphate | 16 | 5.5 |
| Calcium carbonate | 11 | 3.8 |
| Zincstearate | 17 | 6.1 |
| Silica | 18 | 6.2 |
| Peppermint aroma | 0.8 mg | 0.3 |

Ingredients of pharmaceutical quality were used. Colostrum of cows was taken as base. It was purified to remove the caseins and fat using methods known in the art. The whey fraction was purified via cation exchange chromatography and the basic fraction was sterile filtrated and spraydried as described in the art.

The whey fraction comprised (bovine) lactoferrin, lysozyme and lactoperoxidase. The degree of iron saturation of the isolated lactoferrin was below 30%.

Amounts of the ingredients as given above for batches of 400 kg were mixed until a homogeneous mixture was obtained. The mixture was transferred to a standard tabletting machine, which was operated under such a pressure to obtain hard tablets. The pH of tablet was about 7.9.

The tablet can be used three times daily after the meal.

| C. Antiviral/antifungal tablet: | |
|---|---|
| Ingredient | Amount per tablet |
| Conalbumin | 300 mg |
| Lysozyme (from egg white) | 50 mg (about 2.5 * 10 6 IU) |
| Catalase (from bovine liver) | 50 mg (about 150.000 IU) |
| Magnesium stearate | 18 |
| Zinc citrate | 40 |
| Disodium hydrogenphosphate | 100 |
| Potassium hydrogenphosphate | 80 |
| Silica | 18 |
| Peppermint aroma | 1 |
| Peppermint | 40 |
| Aspartame | 2 |
| Sorbitol | 60 |

The manufacturing process as described in example B was applied.

| D. Antiviral/antifungal gel | |
|---|---|
| Ingredient | Amount per 100 g gel |
| Colostrum (WPI) extract | 10 g |
| Sucrose | 10 |
| Gellan gum | 1 |
| Magnesium citrate | 0.5 |
| Zinc citrate | 0.3 |
| Calcium citrate | 0.2 |
| Potassiumhydrogenphosphate | 1 |
| Sodium hydrogen carbonate | 0.3 |
| Fruit aroma | 0.6 |
| Water | about 77 |

Cows were hyperimmunized with a cocktail of viruses (CMV, HIV, HERPES) and fungi (Candida) using known methods. Milk of the first 3 days (colostrum) were used as base ingredient. It was purified to remove the caseins and fat using methods known in the art. The whey fraction was purified via cation-exchange chromatography and the basic fraction was sterile filtrated and spraydried as described in the art.

The gellan gum, sucrose, citrate and phosphate were dissolved in boiling water; after cooling to 65°C the colostrum powder was added. The solution was cooled and the gel was cut into small cubes. Pieces gel (dices) can be kept in the mouth under the tongue for the time required.

E and F. Tablets viruses or fungi

In the composition as given for the tablet of example 2, conalbumin was replaced by 30 mg protegrin PG-1 or 40 mg defensin HNP-1.

| G. Cream | |
|---|---|
| Ingredient | Amount per 100 g |
| Alcohol cetylicus | 15 g |
| Cera alba | 1 |
| Propyleneglycolum | 10 |
| Natrii laurylsulfas | 2 |
| Lactoferricin | 0.2 |

(continued)

| G. Cream | |
|---|---|
| Ingredient | Amount per 100 g |
| $Na_2HPO4$ | 6.8 |
| Citric acid | 0.15 |
| Aqua | about 65 ml |

The ingredients are thoroughly mixed at 50°C until all ingredients have dissolved and the suspension is homogenous. The cream can be applied to the tissues that are affected by Candida albicans.

In these examples B to G, a buffer system was employed as in example A, i.e. $Na_2HPO_4$ 0.2 M and citric acid 0.1 M, topped up with water to 65 ml.

**3. Synergistic fungicidal effect of lactoferrin in combination with antimycotica against clinical isolates of candida**

Introduction

Because of the rising incidence of failures in the treatment of oropharyngeal candidosis in the case of severely immunosuppressed, mostly HIV-infected patients, there is need for the development of new therapeutic compounds which augment the activity of the common antifungal agents. The antifungal effects of human, bovine and iron-depleted lactoferrin in combination with fluconazole, amphotericin B and 5-fluorocytosine in vitro against clinical isolates of Candida species, were investigated.

In a 96-wells plate appropriate concentrations of antifungals were added to an inoculum of Candida, and the minimal inhibitory concentration (MIC) of each antifungal was determined after incubation for 24 hours at 35°C in air. For the combined effects of lactoferrin and the other antifungals a dilution matrix with 8 fold drug dilutions was prepared and synergistic or antagonistic antifungal activities were calculated.

Distinct antifungal activities of lactoferrin were observed against clinical isolates of Candida. The MIC values generally were determined to be in the range of 0.5 to 100 $mg.ml^{-1}$. Interestingly, in the combination experiments pronounced cooperative activity was unexpectedly observed against the growth of Candida using lactoferrin and the three antifungals tested. The use of lactoferrin and fluconazole appeared to be the most successful combination. Significant reductions in minimal effective concentrations of fluconazole were found if combined with relatively small lactoferrin amounts. Such combinations still resulted in complete growth inhibition and synergy up to 50% was noticed against several Candida species.

In the present case the antifungal effect of lactoferrin as combined with some common antifungal agents against several clinical isolates of Candida, was investigated in vitro. Potential cooperative or synergistic anti-Candida activity between lactoferrin and antifungals enable a lower dose of antimycotica during antimycotic therapy.

Organisms

Several oral Candida albicans, C. glabrata an C. tropicalis isolates, that differ in their susceptibility to antifungal agents, were obtained from the routine microbiology services of the Academic Hospital Groningen, The Netherlands. C. albicans ATCC 10231 was used as a control in all susceptibility tests. All strains were stored on Sabouraud dextrose agar slopes at 4°C. (SDA; Oxiod, Unipath Ltd, U.K.)

Assay media

The antifungal agent free media used, Sabouraud Liquid Media (SLM; Oxoid, Unipath Ltd, UK, pH 5.6) and RPMI 1640 medium (with L-glutamine w/o $NaHCO_3$ supplemented with 2% glucose, pH 7.0, Gibco BRL, Paisley Scotland), were prepared according to manufacturers instructions.

Antifungal agents

Bovine lactoferrin, human lactoferrin (both Numico B.V. Wageningen, The Netherlands), fluconazole (Diflucan® I.V.; Pfizer B.V., Holland) and 5-fluorocytosine (Ancotil®, Roche Nederland B.V., Mijndrecht, Holland) were dissolved in assay medium in appropriate concentrations. Apo-lactoferrin was prepared from bovine lactoferrin by overnight dialysis against 0.1 M citric acid according to the method earlier described by Masson, P.L. and Heremans, J.F.

Metal-combining properties of Human Lactoferrin (Red Milk Protein) The Involvement of Bicarbonate in the reaction. Eur. J. Biochem. 6 (1968) 579-584, and handled likewise. Amphotericin B (Fungizone®, Bristol-Myers Squibb Company, Woerden, The Netherlands), was prepared to a concentration of 5 mg/ml in sterile water and was further diluted in assay medium. All suspensions were prepared in sterile glass tubes before addition to the microtitre plate.

In order to exclude the antifungal activity of endotoxins present in the lactoferrin preparations, the anti-Candida effect of lipopolysaccharide alone (LPS, Biowhittaker, Inc. Walkerville, MD, USA) was tested in a range of 1-1000 pg/ml in our assay system as well.

Inoculum

Yeast isolates were grown on SDA for 24 hours at 35°C in air. Suspensions were made by picking 5 colonies from these cultures. These were suspended in 10 ml SLM, and mixed while incubating for 18 hours at 35°C in air. From this culture, a 1:10 dilution in SLM was incubated for 5 hours, resulting in a culture in its growth phase. The latter suspension was also mixed while incubating. This was vortexed, and the turbidity adjusted to a density of a 0.5 McFarland barium sulfate turbidity standard at 530nm, resulted in a concentration of $1\times10^6$-$5\times10^6$ cells per ml. From this, the test inoculum was prepared to a concentration of $1\times10^4$ -$5\times10^4$ cells per ml, by a 1:100 dilution in SLM. Confirmation of the inoculum size was determined using the Spiral Plater, Model C (Spiral Systems, Inc, Cincinnati, Ohio, USA). 100 µl was automatically plated out onto a plate containing SDA, which was incubated for 18 hours at 35°C in air, and the concentration calculated according to the manufacturers specifications.

Assay format

To a sterile 96-well plastic assay plate, flat bottom with matching covers (Corning Costar, Cambridge, U.K.), 50 µl of test inoculum was added. Appropriate concentrations of the antifungal agents to be tested were added to the wells (75-150 µl). Controls were included for the determination of growth habits of each Candida species without the presence of an antifungal agent. The final volume per well was adjusted to 200 µl with the assay medium used (SLM or RPMI).

Incubation, growth curves and endpoint criteria

After inoculation, plates were incubated for 48 hours at 35°C in air, without agitation. Turbidity measurements were performed at t=0 hours, t=18-24 hours (hourly), and 48 hours, at 630 nm in an automated microplate reader ($El_x$800, Bio-Tek Instruments, Inc, Winooski, VT, USA), after resuspending contents of the wells with a multi-channel pipette. Any bubbles were removed with the tip of a sterile needle. Any wells not producing visually or spectrophotometrically positive growth after 48 hours, were confirmed by inoculating 20 µl of the wells contents onto SDA, which were subsequently incubated for 5 days at 35°C in air.

All experiments were performed in quadruplicate.

Synergy experiments

The combined effects of bovine lactoferrin and fluconazole, amphotericin B and 5-fluorocytosine against the growth of Candida species were examined under the experimental conditions as used for determination of the MIC values. A dilution matrix (8 by 8) with 8 fold drug dilutions was prepared. On the basis of three-dimensional surface diagrams, percentages of synergy and antagonism were calculated according to the method described by Prichard, M.N. and Shipman, C., Jr. A three-dimensional model to analyze drug-drug interactions. Antiviral Res. 14 (1990) 181-206. In brief: The theoretical additive effects of a combination of two antifungals from the dose-response values of the individual drugs were calculated. The resulting theoretical dose-response curves were then compared with the actual experimental dose-response curves. For an additive interaction of the two antifungals the actual experimental dose-response curves should coincide with the theoretical ones, but any peaks above or below these values are indicative of synergy or antagonism, respectively.

The synergy experiments were carried out as follows:

All experiments were carried out in four fold, all measured UV values were corrected for the blanco UV values.

As an example, the synergy between fluconazole and lactoferrin against C. glabrata Y110 is provided.

1) A blanco Candida growth was measured in six fold during 24 hours. The Candida growth was measured by way of UV turbidity measurements at 630 nm.

Results: 1.046; 1.217; 1.160; 1.249; 1.212; 1.215

Mean: 1.183

These UV values were subsequently considered as being the maximal Candida growth achievable during the experiments, and were accordingly related to 100%.

2) A dose response curve was made for the individual medicament. The Candida growth was measured in the presence of the medicament. Subsequently the percentage inhibition of the Candida growth by the individual medicament was set out
(inhibition = 100% - growth) with respect to the maximum Candida growth (1.183 = 100%). The results are shown below in Table 3.

Table 3

| | | | also for fluconazole | | |
|---|---|---|---|---|---|
| Lactoferrin (mg/ml) | UV | Inhibition | Fluconazole (mg/ml) | UV | Inhibition |
| 100 | 0.002 | 99.80 | 0.5 | 0.004 | 99.61 |
| 75 | 0.011 | 98.92 | 0.33 | 0.003 | 99.76 |
| 50 | 0.068 | 93.39 | 0.2 | 0.003 | 99.76 |
| 25 | 0.232 | 77.28 | 0.1 | 0.618 | 39.47 |
| 10 | 0.669 | 34.48 | 0.033 | 1.127 | 0.00 |
| 5 | 0.647 | 36.68 | 0.01 | 1.154 | 0.00 |
| 1 | 0.927 | 9.21 | 0.0066 | 1.167 | 0.00 |
| 0.5 | 1.122 | 0.00 | 0.0033 | 1.150 | 0.00 |

3) Subsequently the effect of combinations of lactoferrin and fluconazole against the Candida growth was measured by testing 8 x 8 differing medicament dilutions against one another, whereafter the results with the aid UV measurements and thereafter percentage inhibition were compared.

Experimental

Step 1: the UV measurements of the 8 x 8 dilutions (see Table 4). The negative values resulted as a value of the correction of the blanc.

Table 4

| **Flu** / **LF** | **0.5** | **0.33** | **0.2** | **0.1** | **0.033** | **0.01** | **0.0066** | **0.0033** |
|---|---|---|---|---|---|---|---|---|
| **100** | 0.000 | 0.002 | 0.000 | -0.002 | 0.009 | 0.000 | 0.000 | 0.003 |
| **75** | -0.003 | -0.004 | 0.001 | -0.003 | 0.001 | 0.008 | 0.005 | 0.008 |
| **50** | -0.004 | -0.003 | -0.002 | -0.003 | 0.005 | 0.016 | 0.017 | 0.032 |
| **25** | -0.005 | -0.004 | -0.004 | -0.003 | 0.010 | 0.060 | 0.084 | 0.116 |
| **10** | -0.013 | -0.012 | -0.009 | -0.007 | 0.054 | 0.321 | 0.429 | 0.508 |
| **5** | -0.008 | -0.008 | -0.005 | -0.004 | 0.190 | 0.503 | 0.570 | 0.616 |
| **1** | -0.015 | -0.015 | -0.014 | -0.002 | 0.336 | 0.673 | 0.722 | 0.746 |
| **0.5** | -0.016 | -0.016 | -0.015 | 0.003 | 0.752 | 0.955 | 1.011 | 0.944 |

Step 2. % inhibition (table 5), maximum Candida growth (1.183 = 100%); (inhibition = 100 - % growth).

Table 5

| **Flu** / **LF** | **0.5** | **0.33** | **0.2** | **0.1** | **0.033** | **0.01** | **0.0066** | **0.0033** |
|---|---|---|---|---|---|---|---|---|
| **100** | 100.00 | 99.83 | 100.00 | 100.17 | 99.24 | 100.00 | 100.00 | 99.75 |
| **75** | 100.21 | 100.30 | 99.96 | 100.21 | 99.96 | 99.28 | 99.54 | 99.28 |
| **50** | 100.34 | 100.25 | 100.17 | 100.25 | 99.58 | 98.65 | 98.56 | 97.30 |
| **25** | 100.38 | 100.30 | 100.30 | 100.21 | 99.20 | 94.97 | 92.94 | 90.24 |

Table 5 (continued)

| Flu / LF | 0.5 | 0.33 | 0.2 | 0.1 | 0.033 | 0.01 | 0.0066 | 0.0033 |
|---|---|---|---|---|---|---|---|---|
| **10** | 101.10 | 101.01 | 100.76 | 100.59 | 95.44 | 72.87 | 63.74 | 57.06 |
| **5** | 100.68 | 100.68 | 100.42 | 100.34 | 83.94 | 57.49 | 51.82 | 47.94 |
| **1** | 101.27 | 101.27 | 101.18 | 100.17 | 71.60 | 43.12 | 38.98 | 36.95 |
| **0.5** | 101.35 | 101.35 | 101.27 | 99.75 | 36.44 | 19.28 | 14.55 | 20.21 |

With these last results, 3-dimensional growth inhibition curves were made, whereby the combined inhibitive effect is viewed from above, as showed for example in figure 8.

Subsequently the percentage inhibition of the Candida growth was calculated with the aid of the method described in Prichard, M.N. and Shipman C.Jr. "A three dimensional model to analyze drug-drug interaction." Antiviral Res. 14 (1990) 181-206, page 99, formula 7. It was presumed that lactoferrin and fluconazole have differing working mechanisms. In this way they can be combined as follows:

$$Z=X+Y(1-X) \rightarrow Z=X+Y(100-X/100) \qquad \text{(formula 7)}$$

(wherein it should be noted that the values of X and Y are %).

**Z** = total inhibition caused by the combination of medicament X (lactoferrin) and Y (fluconazole) ;
**X** = inhibition caused by medicament X only (lactoferrin);
**Y** = inhibition caused by medicament Y only (fluconazole).

Step 1

Table 6

| Flu / LF | 0.5 | 0.33 | 0.2 | 0.1 | 0.033 | 0.01 | 0.0066 | 0.0033 |
|---|---|---|---|---|---|---|---|---|
| **100** | 100.00 | 100.00 | 100.00 | 99.88 | 99.80 | 99.80 | 99.80 | 99.80 |
| **75** | 100.00 | 100.00 | 100.00 | 99.35 | 98.92 | 98.92 | 98.92 | 98.92 |
| **50** | 99.97 | 99.98 | 99.98 | 96.00 | 93.39 | 93.39 | 93.39 | 93.39 |
| **25** | 99.91 | 99.94 | 99.94 | 86.25 | 77.28 | 77.28 | 77.28 | 77.28 |
| **10** | 99.74 | 99.84 | 99.84 | 60.34 | 34.48 | 34.48 | 34.48 | 34.48 |
| **5** | 99.75 | 99.84 | 99.84 | 61.67 | 36.68 | 36.68 | 36.68 | 36.68 |
| **1** | 99.64 | 99.78 | 99.78 | 45.04 | 9.21 | 9.21 | 9.21 | 9.21 |
| **0.5** | 99.61 | 99.76 | 99.76 | 39.47 | 0.00 | 0.00 | 0.00 | 0.00 |

The experimentally measured % inhibitions (see point 3 step 2), were finally corrected for the Candida growth inhibition (point 4 step 1).
(Table 5 - Table 6)

Step 2

Table 7

| Flu / LF | 0.5 | 0.33 | 0.2 | 0.1 | 0.033 | 0.01 | 0.0066 | 0.0033 |
|---|---|---|---|---|---|---|---|---|
| **100** | 0.00 | -0.17 | 0.00 | 0.29 | -0.56 | 0.20 | 0.20 | -0.06 |

Table 7 (continued)

| Flu / LF | 0.5 | 0.33 | 0.2 | 0.1 | 0.033 | 0.01 | 0.0066 | 0.0033 |
|---|---|---|---|---|---|---|---|---|
| **75** | 0.22 | 0.30 | -0.04 | 0.86 | 1.04 | 0.36 | 0.61 | 0.36 |
| **50** | 0.36 | 0.27 | 0.19 | 4.26 | 6.19 | 5.26 | 5.17 | 3.91 |
| **25** | 0.47 | 0.35 | 0.35 | 13.97 | 21.92 | 17.69 | 15.67 | 12.96 |
| **10** | 1.36 | 1.17 | 0.92 | 40.25 | 60.96 | 38.39 | 29.27 | 22.59 |
| **5** | 0.92 | 0.83 | 0.58 | 38.67 | 47.26 | 20.81 | 15.14 | 11.26 |
| **1** | 1.62 | 1.49 | 1.41 | 55.13 | 62.39 | 33.91 | 29.77 | 27.74 |
| **0.5** | 1.74 | 1.60 | 1.51 | 60.28 | 36.44 | 19.28 | 14.55 | 20.21 |

These values are represented in a three-dimensional curve (figure 9).

pH effect

The results show that under the reaction conditions at a pH 5.0, lactoferrin (Lf) at a concentration of 100 mg/ml was not active, at a pH 6.0 Lf was relatively active at a concentration of 10 mg/ml and at a pH 7.0 Lf was active at a concentration of 5 mg/ml.

In another medium (SAB) this was seen for pH's 5.6, 6.5 and 7.5.

Synergy effect

The results also show that lactoferrin reinforces the working of azoles, especially fluconazole. With an insensitive progeny Y127, the concentration of lactoferrin should be at least 14 mg/l in spittle in order to provide a good synergistic effect for fluconazole, and should be at least 50 mg/ml to yield a good synergistic fungicidal effect. With a sensitive Candida progeny Y111, the concentration of Lf should be more than about 2 mg/ml. At higher concentrations for example 4 mg/ml, a fungicidal synergistic effect also occurred.

Discussion of the results

Inhibition of Candida growth

The antifungal activities of various forms of lactoferrin (bovine, human and bovine apo-lactoferrin) were determined against a diversity of clinical isolates of C. albicans, C. glabrata and C. tropicana and compared to the susceptibility of Candida species to currently used anti-mycotica. The MICs were determined, according to the recommendations by the NCCLS, after 24 hours of incubation of antifungal agent and Candida species and are presented in Table 8.

Table 8. The minimum inhibitory concentrations (MIC)[1] of several antifungal agents against *Candida* species

| Isolate | Species | Lactoferrin (bovine) (mg/ml) | Apo-Lactoferrin (mg/ml) | Amphoterricin B (µg/ml) | Fluconazol (µg/ml) | 5-Fluorocytosine (µg/ml) |
|---|---|---|---|---|---|---|
| 10231[2] | *C. albicans* | 97.4 ± 46 | 21.8 ± 13 | 0.06 ± 0.05 | - | - |
| Y098[3] | *C. albicans* | 20.8 ± 0.96 | 54.3 ± 2.2 | 0.08 ± 0.03 | - | - |
| Y106 | *C. albicans* | 0.5 | 32.9 ± 6.9 | 0.082 ± 0.03 | 10 | - |
| Y127 | *C. albicans* | 97.9 ± 42 | 41.0 ± 11 | 0.15 ± 0.07 | 10 | - |
| Y110 | *C. glabrata* | 30.9 ± 14 | 57.1 ± 6.8 | 0.173 ± 0.06 | 156 ± 50 | - |
| Y111 | *C. glabrata* | 6.3 ± 4.8 | < 5 | 0.14 ± 0.07 | 24 ± 7 | - |
| Y112 | *C. glabrata* | 20.5 ± 8.1 | 41.3 ± 14 | 0.4 ± 0.05 | - | - |
| Y110 (RPMI)[4] | *C. glabrata* | -[5] | - | - | - | 0.029 ± 0.005 |
| Y140 (RPMI)[4] | *C. tropicana* | - | - | - | - | 35 ± 7.07 |

[1] The minimal inhibitory concentration (MIC) was defined as the lowest antifungal agent concentration that substantially inhibited the growth of *Candida* after 24 hours according to the recommendations by the NCCLS for the several antifungal agents used [25]. All experiments were performed in quadruplicate.
[2] Isolate 10231 is an ATCC strain
[3] All Y-isolates are clinical, mostly oral, *Candida* isolates
[4] These isolates were tested in **RPMI-medium instead of SLM**
[5] - means not determined

Since the antifungal activity of human lactoferrin was comparable or even less active as compared to the bovine variant (results not shown), all other experiments were continued with bovine lactoferrin because of its better availability. Bovine lactoferrin and bovine apo-lactoferrin both exhibited equivalent antifungal activities. The MICs found for these two variants were all in the same range and were for the Candida species tested in SLM medium in the range of 0.5 to 100 mg/ml.

Lactoferrin is able to bind lipopolysaccharide (LPS). In an earlier experiment (not detailed) the LPS content of these milk proteins (5 pg/mg protein) was determined. In order to exclude the contribution of LPS to the antifungal activity of these milk proteins, the antifungal activity of LPS in the test system was examined. It was noticed that up to a concentration of 1000 pg/ml LPS no killing of Candida species was observed. Because concentrations of lactoferrin up to 100 mg/ml do contain 500 pg/ml LPS, is was assumed that the inhibition of Candida species is predominantly caused by the milk protein itself.

Combination of fluconazole and lactoferrin

The combined effect of fluconazole and lactoferrin against the growth of the Candida isolate yl10 is shown in Figure 8. It was found that this Candida specie was already completely inhibited in its growth using for example 50 μg/ml fluconazole in combination with 10 mg/ml lactoferrin, whereas their MIC values against this isolate were 159 μg/ml and 39 mg/ml respectively (table 4). This implicated a complete inhibition of Candida growth using less antimycotic as could be extrapolated from their MIC values. This accounts as well for other combinations of lactoferrin and fluconazole (figure 8). Several combinations of fluconazole and lactoferrin resulted in a highly synergistic anti-Candida effects against Y110, as is shown in Figure 9. Effects above baseline from +5% to +50% were observed. For example a combination of 0.5 mg/ml lactoferrin and 100 μg/ml fluconazole resulted in 50% synergistic anti-Candida effects, whereas 25 mg/ml lactoferrin in combination with 3.3 μg/ml fluconazole induced only 5% extra anti-Candida effect. An increase in concentration of fluconazole or lactoferrin (towards their MIC values) demonstrated less synergistic effect. This was expected because a concentration of fluconazole towards its MIC value, is at itself already capable of complete Candida growth inhibition. No antagonistic anti-Candida activity between lactoferrin and fluconazole was observed for this isolate.

Y127, a rather lactoferrin insensitive Candida isolate (MIC value of 100 mg/ml), was completely inhibited using 10 mg/ml lactoferrin in combination with 1 μg/ml fluconazole, while the MIC of fluconazole was 10 μg/ml (Figure 10). In addition antagonistic as well as synergistic Candida growth inhibition was found, in the range from -20% to +40% 20% antagonism to 40% synergism respectively). A combination of 1 mg/ml lactoferrin and 0.05 μg/ml fluconazole resulted in 20% antagonistic effects: 20% less inhibition of Candida growth than theoretically expected on basis of the individual inhibitory effects of lactoferrin and fluconazole, whereas 25 mg/ml lactoferrin in combination with 0.5 μg/ml fluconazole induced as much as 40% extra growth inhibition (Figure 11).

Likewise Y111, one of the more lactoferrin sensitive strains, was efficiently inhibited using combinations of lactoferrin and fluconazole. A reduction by 50% of the MIC values of both compounds against this isolate resulted in a complete inhibition of Candida growth (1 mg/ml lactoferrin with 10 μg/ml fluconazole). The cooperative activity of fluconazole and lactoferrin was as well antagonistic (10%) using of minor amounts of both compounds (0.005 mg/ml lactoferrin and 0.3 μg/ml fluconazole) as well as synergistic (50%) using a concentration of 0.5 mg/ml lactoferrin and 10 μg/ml fluconazole.

Combination of amphotericin B and lactoferrin

Efficient inhibition of the Candida growth was also observed with a the combination of amphotericin B and lactoferrin against isolate Y110. A complete inhibition of the Candida growth was observed using 0.5 mg/ml lactoferrin and 0.1 μg/ml amphotericin B (Figure 12). In addition both antagonistic (10%) and synergistic (30%) inhibition of the Candida growth was demonstrated (Figure 13).

Yet, against isolate Y127 the combination of lactoferrin and amphotericin B resulted in a not so pronounced decrease in sufficient concentrations of lactoferrin or amphotericin B to obtain complete Candida inhibition. Complete inhibition of the Candida growth could only be obtained using concentrations of amphotericin B or lactoferrin close towards their MIC values. Still antagonistic (10%) as well as synergistic effects (30%) of this combination against Y127 could be observed. 30% synergistic effects were detected using 30 mg/ml lactoferrin in combination with 0.1 μg/ml amphotericin-B, which is only a small decrease as compared to the MIC value of amphotericin B itself (MIC value: 0.15 μg/ml).

Combination of 5-fluorocytosine and lactoferrin

A combination of 5-fluorocytosine and lactoferrin resulted in an effective inhibition of the growth of isolate

Y110. 100% growth inhibition was observed using 0.0008 μg/ml 5-fluorocytosine (a decline of 30 times as compared to its MIC value) in combination with 0.02 mg/ml lactoferrin. This combination of antifungals did demonstrate synergistic activity against Y110 of 50% using 0.025 μg/ml 5-fluorocytosine in combination with 0.01 mg/ml lactoferrin. Yet, also minor antagonistic effects of 5% on the growth inhibition were observed using 0.001 μg/ml 5-fluorocytosine in combination with 0.01 mg/ml lactoferrin.

The C. tropicalis isolate Y140 is a 5-fluorocytosine resistant isolate (see also table 4). The antifungal effects against Y140 only using a combination of 5-fluorocytosine and lactoferrin were investigated, wherein 90% inhibition was reached. Synergistic effects of 15% using 10 mg/ml lactoferrin and 10 μg/ml 5-fluorocytosine and antagonistic effects of 10% using 0.001 mg/ml lactoferrin and 45 μg/ml 5-fluorocytosine were seen.

The influence of saliva on the antifungal activity of lactoferrin against candida species, was investigated.

Materials and methods

Organisms

Candida glabrata yl10 was obtained from the routine microbiology services of the Academic Hospital Groningen, The Netherlands, and was stored on Sabouraud dextrose agar slopes at 4°C. (SDA; Oxiod, Unipath Ltd, U.K.)

Assay media

The antifungal agent free media used, Sabouraud Liquid Media (SLM; Oxoid, Unipath Ltd, UK, pH 5.6) and RPMI 1640 medium (with L-glutamine w/o $NaHCO_3$ supplemented with 2% glucose, pH 7.0, Gibco BRL, Paisley Scotland), were prepared according to manufacturers instructions. The pH of SLM or RPMI was adjusted to 7.5 using NaOH. The media were sterilized by filtering through 0.2 μm filters.

Saliva

Saliva was collected from human volunteers. After centrifugation for 10 min at 2000 x g, the pH of saliva was measured. The saliva was sterilized by filtering through 0.2 μm filters and stored at -20°C until use. Saliva was added to assay media (1:1) (RPMI and SLM), which were twice the normal concentration, resulting in normal concentrations of RPMI and SLM.

Antifungal agents

Bovine lactoferrin (Numico B.V. Wageningen, The Netherlands) and fluconazole (Diflucan® I.V.; Pfizer B.V., Holland) were dissolved in assay medium in appropriate concentrations. All suspensions were prepared in sterile glass tubes before addition to the microtitre plate.

Inoculum

The yeast isolates were grown on SDA for 24 hours at 35°C in air. Suspensions were made by picking 5 colonies from these cultures. These were suspended in 10 ml SLM, and mixed while incubating for 18 hours at 35°C in air. From this culture, a 1:10 dilution in either SLM or RPMI (pH 7.5) was incubated and mixed for 5 hours, resulting in a culture in its growth phase. This was vortexed, and the turbidity adjusted to a density of a 0.5 McFarland barium sulfate turbidity standard at 530nm, resulted in a concentration of $1\times10^6$-$5\times10^6$ cells per ml as previously described. From this, the test inoculum was prepared to a concentration of $1\times10^4$ -$5\times10^4$ cells per ml, by a 1:100 dilution in SLM. Confirmation of the inoculum size was determined using the Spiral Plater, Model C (Spiral Systems, Inc, Cincinnati, Ohio, USA). 100 μl was automatically plated out onto a plate containing SDA, which was incubated for 18 hours at 35°C in air, and the concentration calculated according to the manufacturers specifications.

Assay format

To a sterile 96-well plastic assay plate, flat bottom with matching covers (Corning Costar, Cambridge, U.K.), 50 μl of test inoculum was added. Appropriate concentrations of the to be tested antifungal agents were added to the wells (75-150 μl). Controls were included for the determination of growth habits of each Candida species without the presence of an antifungal agent. The final volume per well was adjusted to 200 μl with the assay medium used (SLM or RPMI).

Incubation, growth curves and endpoint criteria

After inoculation, plates were incubated for 48 hours at 35°C in air, without agitation. Turbidity measurements were performed at t=0 hours, t=18-24 hours (hourly), and 48 hours, at 630 nm in an automated microplate reader ($El_x$800, Bio-Tek Instruments, Inc, Winooski, VT, USA), after resuspending contents of the wells with a multi-channel pipette. Any bubbles were removed with the tip of a sterile needle. Any wells not producing visually or spectrophotometrically positive growth after 48 hours, were confirmed by inoculating 20 µl of the wells contents onto SDA, which were subsequently incubated for 5 days at 35°C in air.
The minimal inhibitory concentration (MIC) was defined as the lowest antifungal agent concentration that substantially inhibited the growth of Candida after 24 hours according to the recommendations by the NCCLS for the antifungal agents used. All experiments were performed in quadruplicate.

Results

The effect of saliva on the growth of Candida species

The addition of saliva to the assay medium SLM resulted in a small delay in growth rate of the Candida glabrata Y110, as shown in figure 22. However, after 24 hours of incubation the amount of Candida species present was similar. On the other hand the addition of saliva to the assay medium RPMI resulted in an almost complete inhibition of the Candida growth (figure 23 (a, b, c)). Only the addition of 5% saliva showed some Candida growth, as compared to the control (0% saliva). We therefore used for the other experiments saliva in combination with the SLM medium (50%/ 50%).

The inhibition of the Candida growth in the presence of saliva

Lactoferrin - Y110

A concentration range of lactoferrin was incubated for 24 hours with C. glabrata Y110 in the presence of saliva. As can be seen in figure 24 and 25, the growth rate of the Candida was delayed in the presence of saliva. An effect already observed in the control experiment (figure 1). Lactoferrin was however still able to inhibit efficiently the growth of the Candida isolate. The delay in growth of the Candida isolate in the presence of the saliva was as well reflected in the MIC values. The MIC for lactoferrin tested in SLM was 11.7 mg/ml, whereas in SLM/Saliva the MIC was somewhat lower (9.6 mg/ml). This could be expected since a slower growing fungus is earlier to inhibit in its growth. In addition components of saliva itself can be capable of inhibition of the Candida.

Fluconazole - Y110

The antifungal activity of fluconazole exhibit a similar pattern against Y110 in the presence of saliva as compared to the activity of lactoferrin as described above (figure 26(a-h) and 27(a-h)). The MIC values of fluconazole were 0.1 mg/ml in SLM and 0.04 mg/ml in SLM/Saliva.

Combination of fluconazole and lactoferrin in the presence of saliva

The combined effect of fluconazole and lactoferrin against the growth of the Candida isolate Y110 in SLM at pH 7.5 is shown in Figure 28. It was found that this Candida specie was already completely inhibited in its growth using for example 50 µg/ml fluconazole in combination with 0.5 mg/ml lactoferrin, whereas their MIC values against this isolate were 100 µg/ml and 11 mg/ml respectively (see above). This implicates a complete inhibition of Candida growth using less antimycotic as could be extrapolated from their MIC values. This accounts as well for other combinations of lactoferrin and fluconazole (figure 28). Several combinations of fluconazole and lactoferrin resulted in a highly synergistic anti-Candida effects against Y110, as is shown in Figure 29. Effects above baseline from ±5% to ±50% were observed. For example a combination of 0.5 mg/ml lactoferrin and 33 µg/ml fluconazole resulted in 50% synergistic anti-Candida effects, whereas 25 mg/ml lactoferrin in combination with 33 µg/ml fluconazole induced only 5% extra anti-Candida effect. An increase in concentration of fluconazole or lactoferrin (towards their MIC values) demonstrated less synergistic effect. This was expected because a concentration of fluconazole towards its MIC value, is at itself already capable of complete Candida growth inhibition. No antagonistic anti-Candida activity between lactoferrin and fluconazole was observed for this isolate.
The combined addition of fluconazole and lactoferrin to Y110 in the presence of saliva (50%) resulted in a shift of complete inhibition towards lower concentrations of antifungals (figure 30). Although the MIC values for both

antifungals against Y110 in SLM/Saliva were lower as compared to the MIC values in normal SLM medium (see above), still the combination of both compounds resulted in a far more effective inhibition of the growth of this Candida isolate. For example a combination of 0.01 mg/ml lactoferrin and 3 µg/ml fluconazole exhibited a complete inhibition of the Candida growth in SLM/Saliva medium, whereas in normal SLM medium this combination only accounted for 11% inhibition.

The synergistic antifungal activity of this combination against Y110 incubated in SLM/Saliva is shown in figure 31. As compared to the synergistic effects observed in SLM alone, the addition of saliva to the incubation medium resulted in an increase in the amount of synergistic combinations of lactoferrin and fluconazole. Effect above baseline of 65% were observed using a combination of 1 µg/ml lactoferrin and 33 µg/ml fluconazole. On the other hand antagonistic effects of 30% were observed using a combination of 10 µg/ml lactoferrin and 0.1 µg/ml fluconazole.

The invention is not limited to the above description; the requested rights are rather determined by the following claims.

## Claims

**1.** Medicament for treatment and/or preventment of infections caused by bacteria, fungi, viri and the like, inflammations and/or tumors, said medicament comprising an active amount of a polycationic peptide or protein, and a buffer for maintaining the pH of treatable tissue within a preselected range.

**2.** Medicament according to claim 1, wherein the buffer maintains the pH of treatable tissue in the range of about 5 to 8.5, and preferably maintains a pH of treatable tissue in the range of between about 7 and 8.

**3.** Medicament according to claims 1 or 2, wherein the polycationic peptide or protein is selected from the group consisting essentially of:

- human lactoferrin, bovine lactoferrin, lactoferricin, conalbumin (ovotransferrin), the polycationic peptides occurring in these proteins, hydrolysates of lactoferrin, and cation rich peptides originating from lactoferrin;
- poly-peptide having an amino acid sequence selected from the following sequences (1)-(15), or derivatives thereof having an amide at the carboxy end thereof:

(1) Arg-Trp-Gln-Trp-Arg;
(2) Arg-Arg-Gln-Trp-Arg;
(3) Lys-Val-Ser-Trp-Arg;
(4) Arg-Asn-Met-Arg-Lys;
(5) Arg-Trp-Gln-Glu-Lys;
(6) Arg-Arg-Trp-Gln-Trp-Arg;
(7) Arg-Arg-Arg-Gln-Trp-Arg;
(8) Lys-Thr-Val-Ser-Trp-Arg;
(9) Lys-Arg-Asn-Met-Arg-Lys;
(10) Arg-Trp-Gln-Glu-Met-Lys;
(11) Lys-Thr-Arg-Arg-Trp-Gln-Trp-Arg-Met-Lys-Lys;
(12) Lys-Ser-Arg-Arg-Arg-Gln-Trp-Arg-Met-Lys-Lys;
(13) Lys-Thr-Val-Ser-Trp-Gln-Thr-Tyr-Met-Lys-Lys;
(14) Lys-Thr-Phe-Gln-Trp-Gln-Arg-Asn-Met-Arg-Lys;

(15) Lys-Thr-Leu-Arg-Trp-Gln-Asn-Glu-Met-Arg-Lys;

a peptide containing one of the following amino acid sequences (a), (b), (c), or (d) :

S——S

-Lys-Cys-Arg-Arg-Trp-Gln-Trp-Arg-Met-Lys-Lys-Leu-Gly-Ala-

Pro-Ser-Ile-Thr-Cys-Val-: (a)

-Lys-Cys*-Arg-Arg-Trp-Gln-Trp-Arg-Met-Lys-Lys-Leu-Gly-
Ala-Pro-Ser-Ile-Thr-Cys*-Val-: (b)

S——S

-Lys-Cys-Phe-Gln-Trp-Gln-Arg-Asn-Met-Arg-Lys-Val-Arg-Gly-

Pro-Pro-Val-Ser-Cys-Ile-: (c)

- Lys-Cys*-Phe-Gln-Trp-Gln-Arg-Asn-Met-Arg-Lys-Val-Gly-Pro-Pro-Val-Ser-Cys*-Ile-: (d)

where Cys* represents cysteine in which the thiol group is blocked in order to prevent disulfide bond formation; and mixtures thereof and pharmaceutically and sitologically acceptable salts thereof;

- a peptide consisting of one of the following specific amino acid sequences (a)-(1) or derivatives thereof having an amide at the carboxy end thereof:

(a) Phe-Gln-Trp-Gln-Arg-Asn
(b) Phe-Gln-Trp-Gln-Arg
(c) Gln-Trp-Gln-Arg
(d) Trp-Gln-Arg
(e) Arg-Arg-Trp-Gln-Trp
(f) Arg-Arg-Trp-Gln
(g) Trp-Gln-Trp-Arg
(h) Gln-Trp-Arg
(i) Leu-Arg-Trp-Gln-Asn-Asp
(j) Leu-Arg-Trp-Gln-Asn

(k) Leu-Arg-Trp-Gln
(l) Arg-Trp-Gln,

and lactoferrin hydrolyzate for the manufacture of antibacterial agent, and chemical derivatives thereof, wherein by the derivatives, the polarity of the amino group of the amino acid residue constituting the protein is chemically modified into a negative moiety;

- polycations belonging to the family of $\alpha$ or $\beta$ defensins, such as magainins, cecropins type A or B, protegrins, indolicidin analogs, polycations isolatable from insects, and histones;
- mixtures thereof; and
- pharmaceutically and cytologically acceptable salts of this group.

**4.** Medicament according to claim 3, wherein the polycationic peptide is lactoferrin.

**5.** Medicament according to any of the preceding claims, wherein the buffer is selected from the group consisting essentially of carbonate, phosphate, tromethamine, and tetrahydroxypropyl ethylenediamine buffers, and/or suitable salts thereof, especially citrate salts.

**6.** Medicament according to any of the preceding claims comprising at least 0.5 μmol, preferably 5 or more μmol polycationic peptide or protein, and wherein the buffer is present in at least 1 μmol, preferably 2 or more μmols.

**7.** Medicament according to any of the previous claims wherein the buffer is present in the range of 0.5-100 meq $H^+$ and preferably 0.8-20 meq $H^+$ per unit dose medicament.

**8.** Medicament according to any of the preceding claims further comprising one or more of the following, standard excipients, diluents and carriers.

**9.** Medicament according to any of the preceding claims, further comprising a standard anti-fungal, antibacterial, and/or antiviral agent, preferably being selected from the group consisting essentially of azole compounds, 5-fluorocytosine, polyenes, for example pimaricine, fungicidine, and amphotericine B, specifically fluconazol, Amphotericin B and 5-fluorocytosine.

**10.** Medicament according to claim 9 wherein the antifungal agent is present in the medicament in the range 0.025 mg - 50 mg, preferably 0.5-5 mg.

**11.** Medicament for treatment and/or prevention of infections caused by bacteria, fungi, viri and the like, inflammations and/or tumors, said medicament comprising a polycationic peptide or protein being present in the medicament at a predetermined level in order to yield a synergistic pharmaceutical effect in combination with separately administerable bacterial, fungal and viral medicaments.

**12.** Medicament of claim 11 wherein the polycationic peptide or protein is selected from the group as defined in claims 3 or 4, and is present in the medicament in an amount of at least 10 mg/ml, for example at least 20 mg/ml, preferably at least 60 mg/ml and most preferably at least 100 mg/ml bodily fluid.

**13.** Medicament according to claim 12, further comprising one or more antifungal agents as defined in claim 9 and/or one or more excipients, diluents or carriers as defined in claim 8.

**14.** Medicament according to claim 13, wherein the anti-fungal agents are present in an amount of at least 0.1 mg/ml, and preferably at least 0.2 mg/ml.

**15.** Medicament according to any of the preceding claims and/or pharmaceutically acceptable salts thereof having one or more of the following forms:
tablet, spray, salve, gel, liquid.

**16.** Composition comprising a polycationic peptide or protein as defined in claims 3 or 4, and a buffer as defined in claim 5.

**17.** Composition comprising a polycationic peptide or protein in a concentration range as defined in claim 10.

**18.** Use of a composition according to claim 16 or 17 for preparing a medicament.

**19.** Use of a composition according to claims 14 or 15 for the treatment and/or prevention of infections, caused by bacteria, fungi and virus and the like inflammations and/or tumors.

**20.** Use of a composition according to claim 19, for combatting candidiasis.

**21.** Use of a composition according to claim 16 or 17 for the manufacture of a medicament for treating infections caused by bacteria, fungi, viri and the like, inflammations and/or tumors specifically candidiasis.

**22.** A method for treatment and/or prevention of infections caused by bacteria, fungi, viri and the like, inflammations and/or tumors whereby an effective amount of a composition according to claims 16 or 17 is administered to a patient.

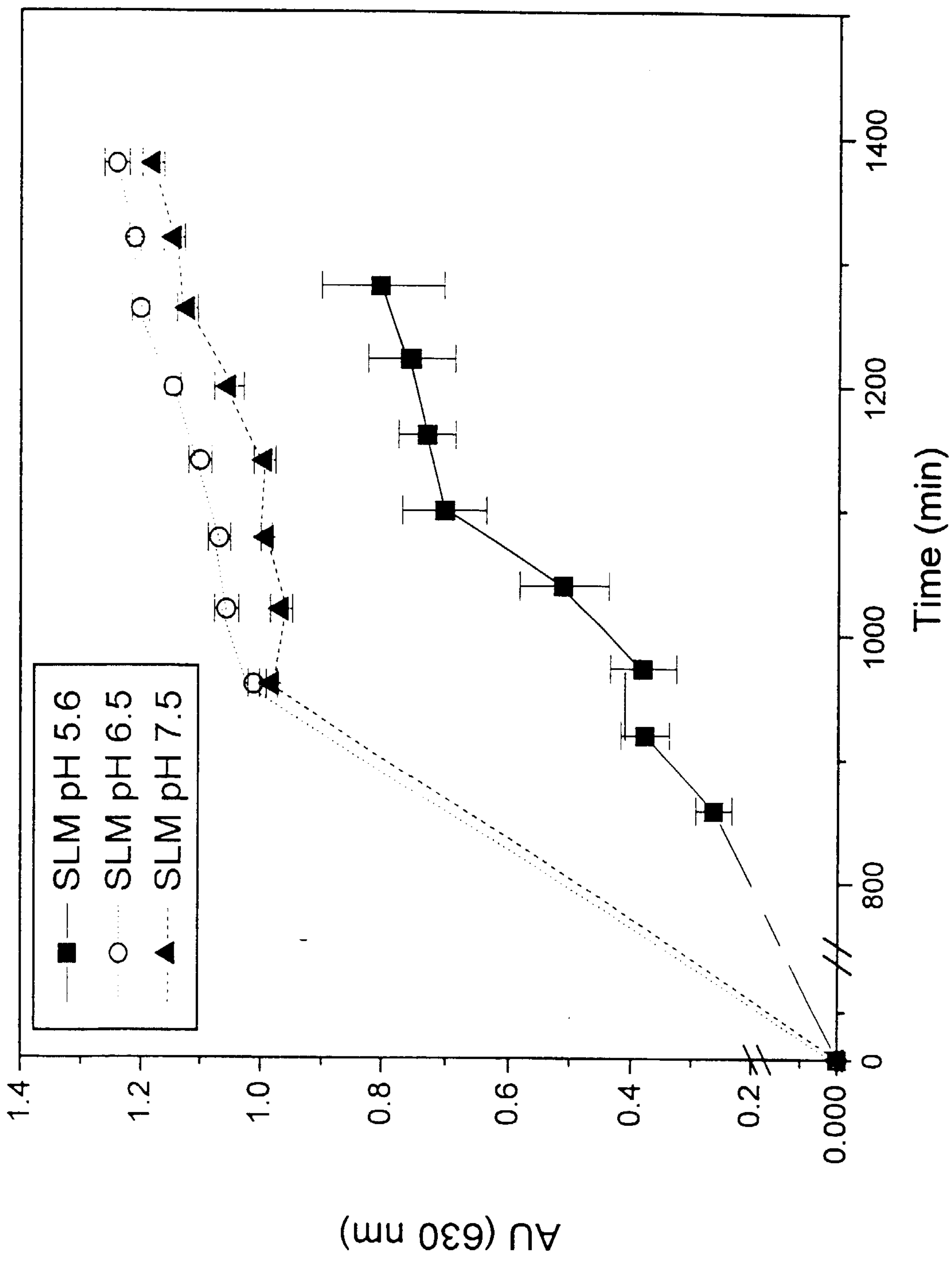
SLM pH 5.6
SLM pH 6.5
SLM pH 7.5
AU (630 nm)
Time (min)
0.000
0.2
0.4
0.6
0.8
1.0
1.2
1.4
0
800
1000
1200
1400

Fig. 1

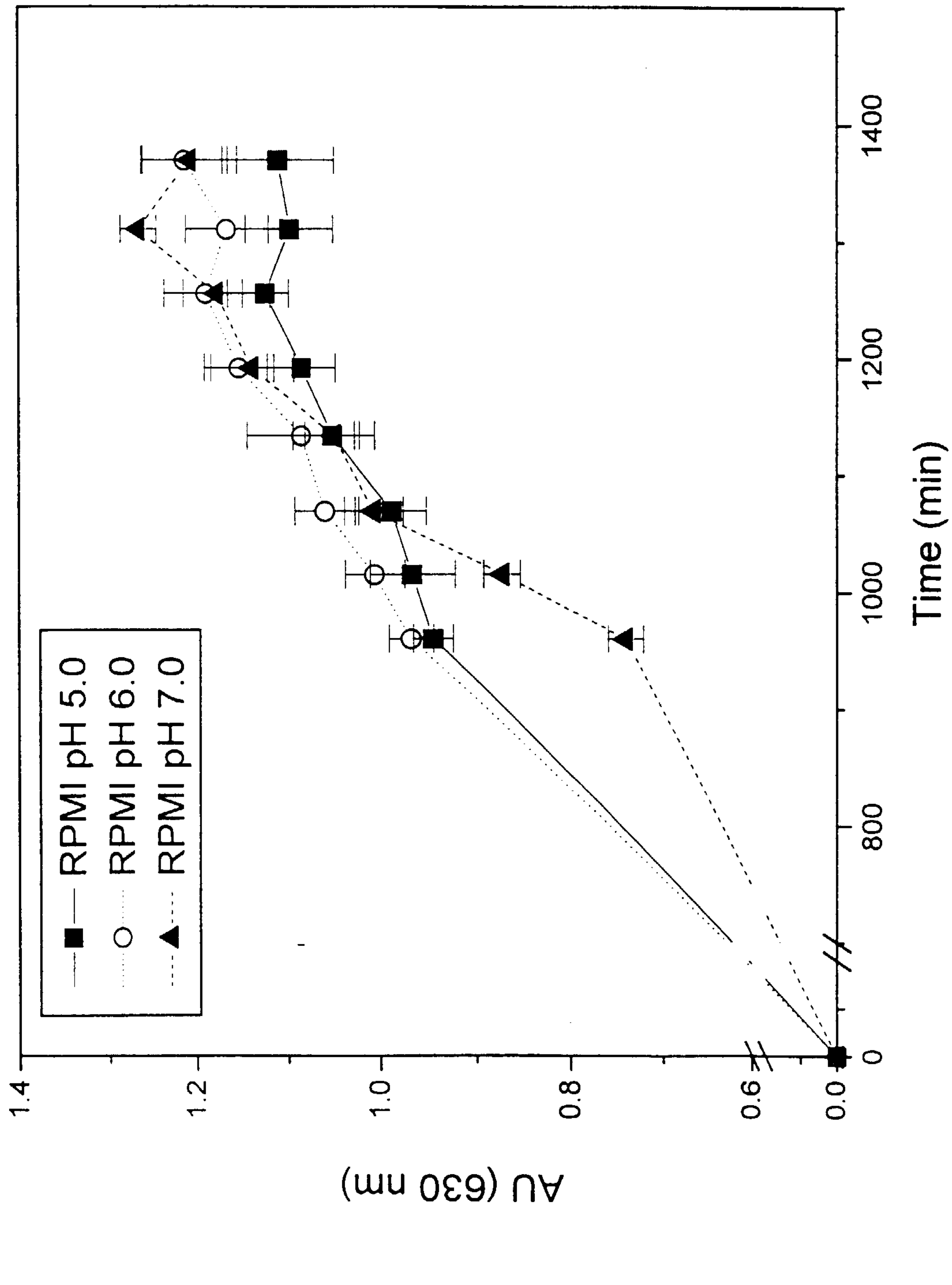
RPMI pH 5.0
RPMI pH 6.0
RPMI pH 7.0
AU (630 nm)
Time (min)
0.0
0.6
0.8
1.0
1.2
1.4
0
800
1000
1200
1400

Fig. 2

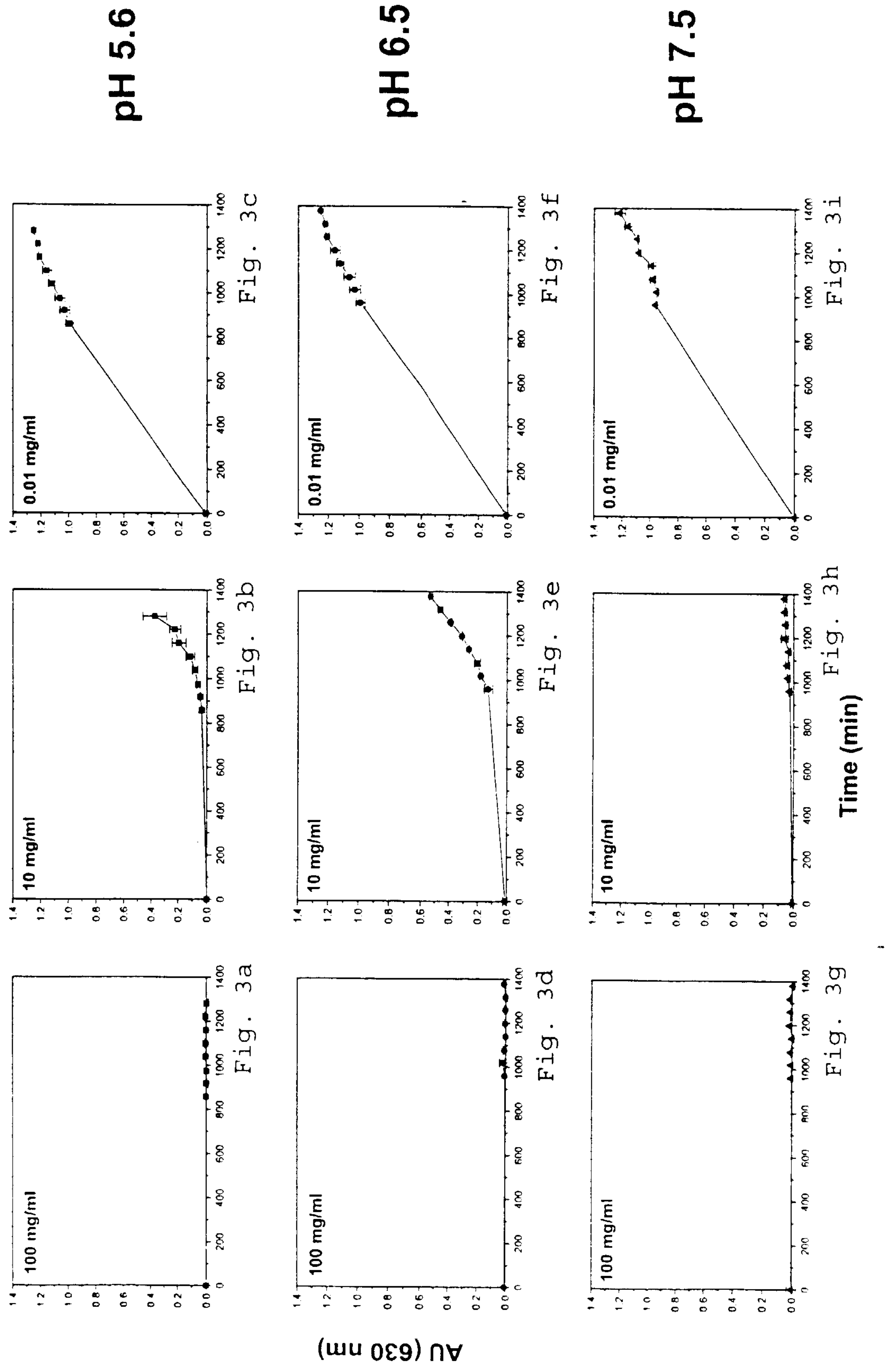
pH 5.6
pH 6.5
pH 7.5
100 mg/ml
10 mg/ml
0.01 mg/ml
AU (630 nm)
Time (min)
Fig. 3a
Fig. 3b
Fig. 3c
Fig. 3d
Fig. 3e
Fig. 3f
Fig. 3g
Fig. 3h
Fig. 3i

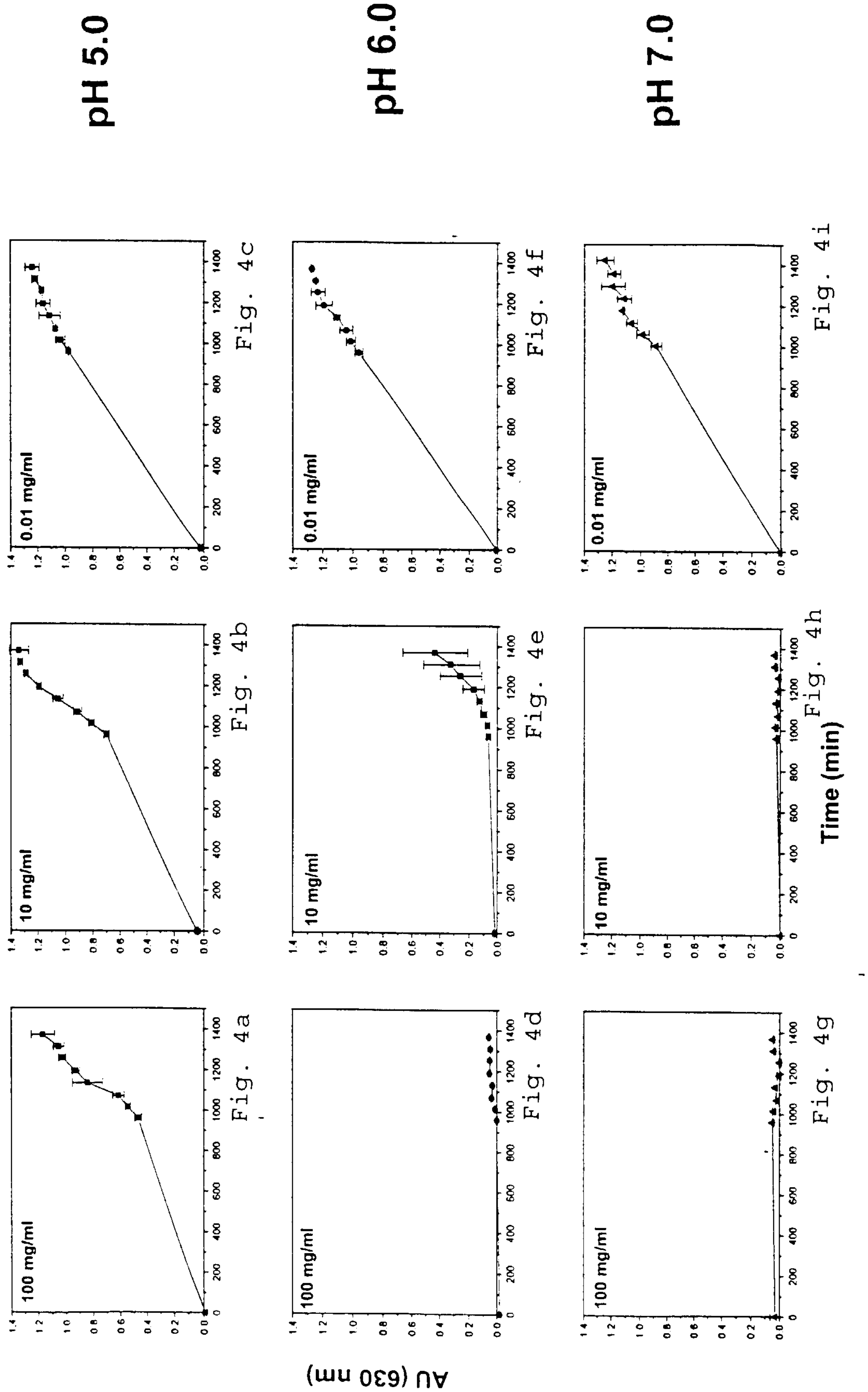


Fig. 4a Fig. 4b Fig. 4c Fig. 4d Fig. 4e Fig. 4f Fig. 4g Fig. 4h Fig. 4i

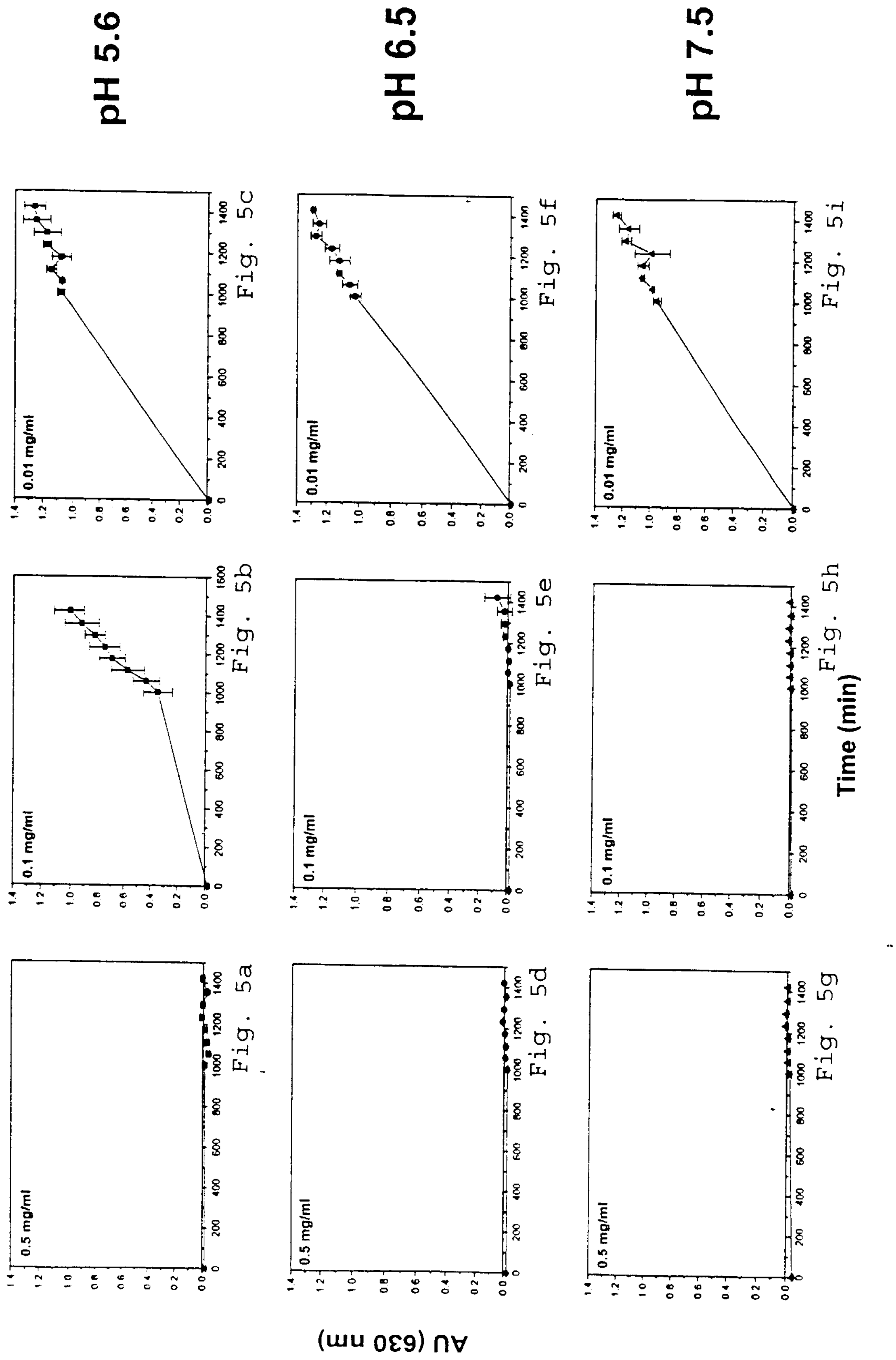


Fig. 5a Fig. 5b Fig. 5c
Fig. 5d Fig. 5e Fig. 5f
Fig. 5g Fig. 5h Fig. 5i

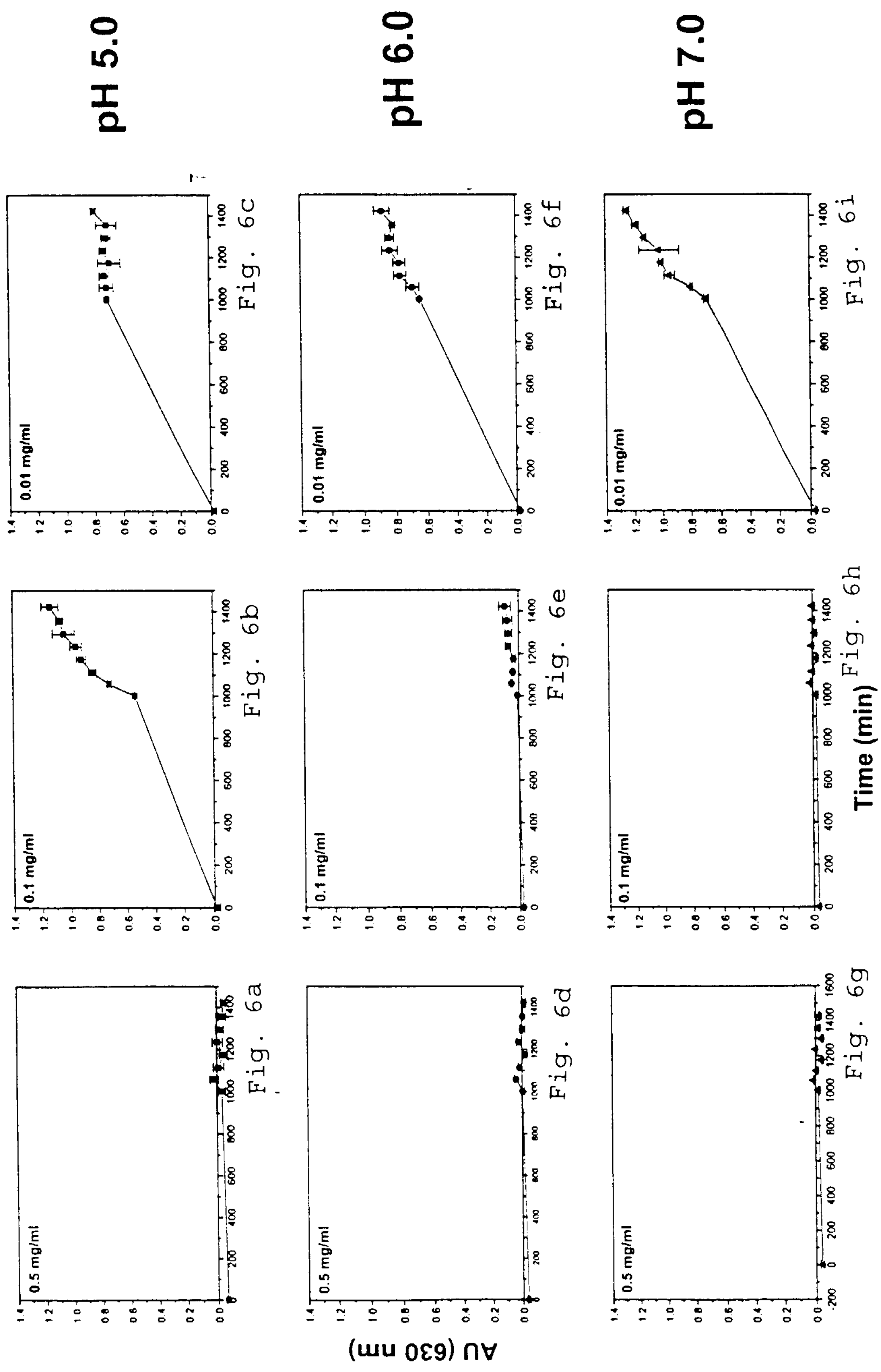

pH 5.0
pH 6.0
pH 7.0
0.5 mg/ml
0.1 mg/ml
0.01 mg/ml
AU (630 nm)
Time (min)
Fig. 6a
Fig. 6b
Fig. 6c
Fig. 6d
Fig. 6e
Fig. 6f
Fig. 6g
Fig. 6h
Fig. 6i

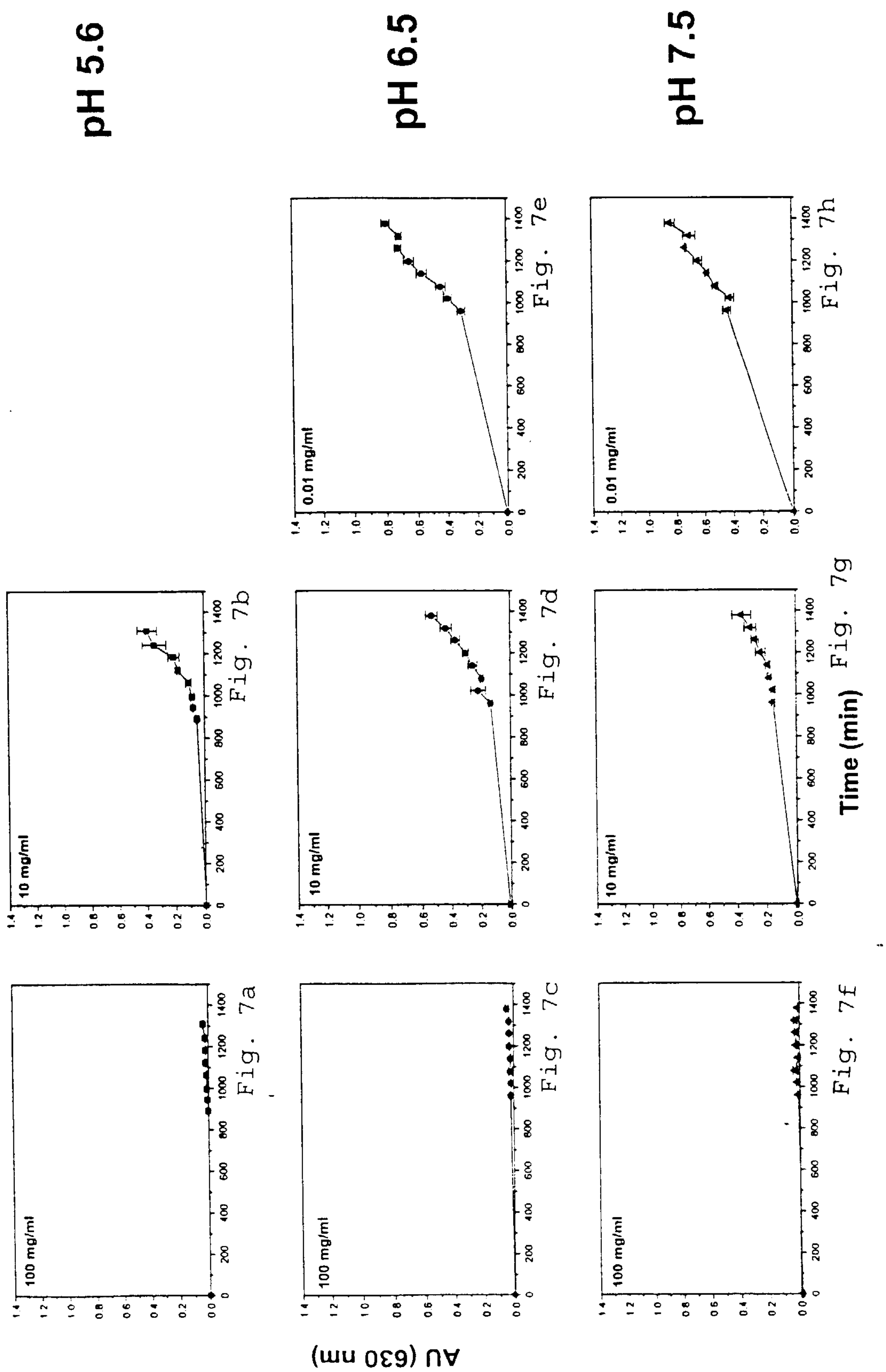
pH 5.6
pH 6.5
pH 7.5
100 mg/ml
10 mg/ml
0.01 mg/ml
AU (630 nm)
Time (min)
Fig. 7a
Fig. 7b
Fig. 7c
Fig. 7d
Fig. 7e
Fig. 7f
Fig. 7g
Fig. 7h

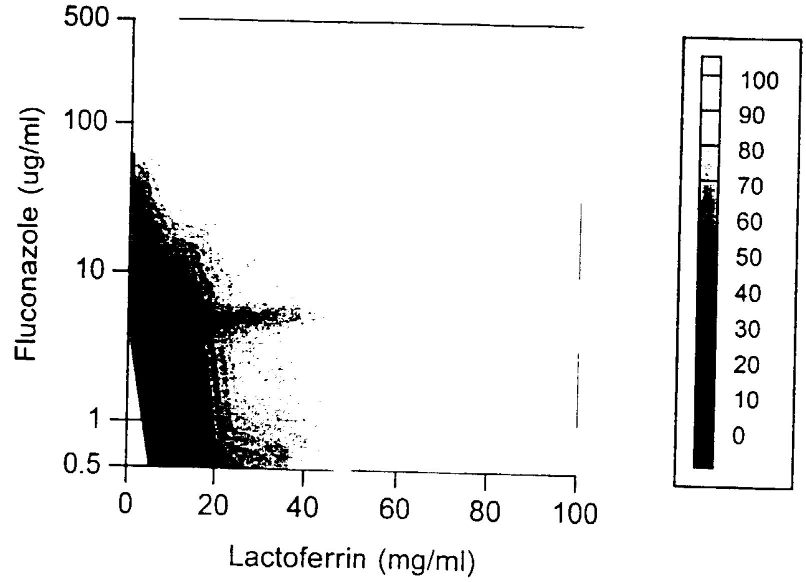
500
100
10
1
0.5
Fluconazole (ug/ml)
0
20
40
60
80
100
Lactoferrin (mg/ml)
100
90
80
70
60
50
40
30
20
10
0

Fig. 8

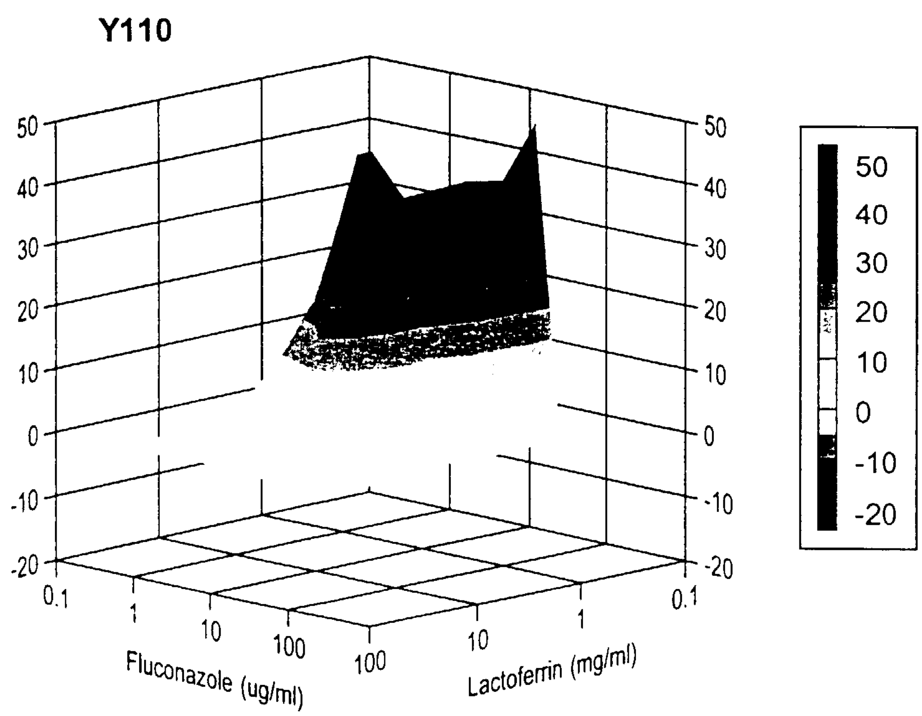
Y110
50
40
30
20
10
0
-10
-20
0.1
1
10
100
Fluconazole (ug/ml)
Lactoferrin (mg/ml)

Fig. 9

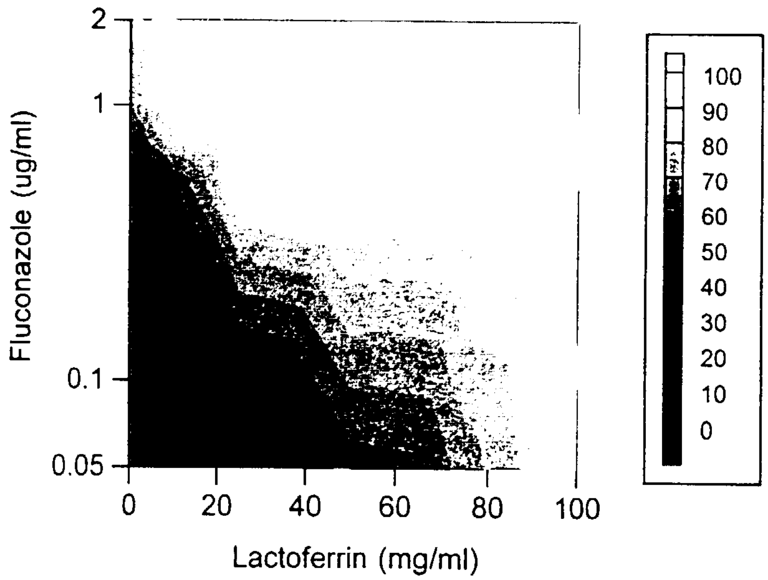
Fluconazole (ug/ml)
2
1
0.1
0.05
0
20
40
60
80
100
Lactoferrin (mg/ml)
100
90
80
70
60
50
40
30
20
10
0

Fig. 10

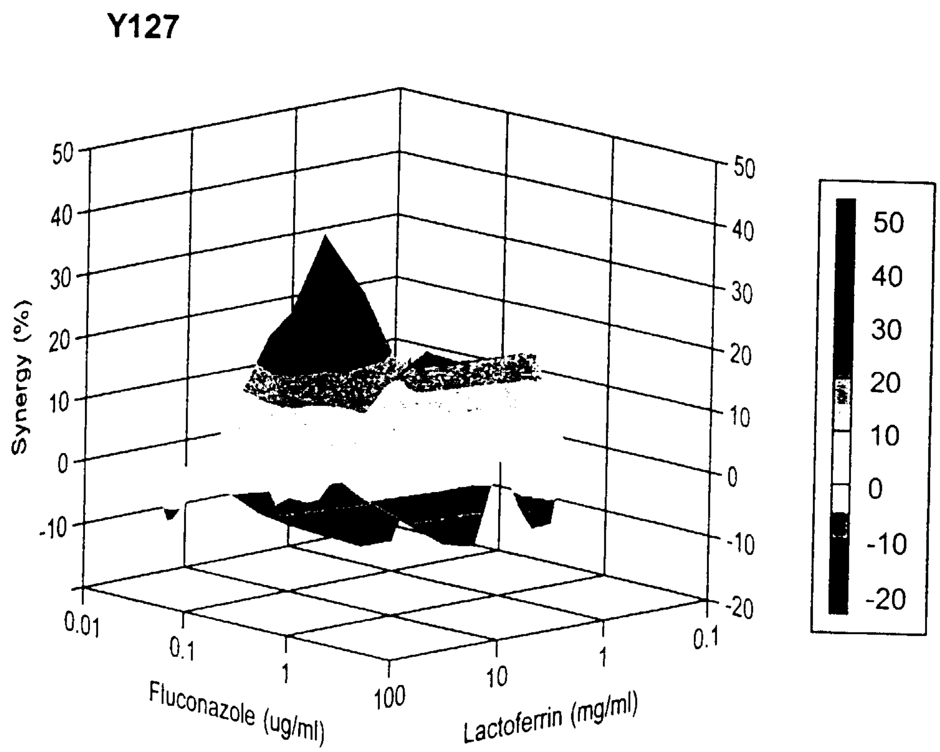
Y127
Synergy (%)
50
40
30
20
10
0
-10
-20
0.01
0.1
1
100
Fluconazole (ug/ml)
10
1
0.1
Lactoferrin (mg/ml)

Fig. 11

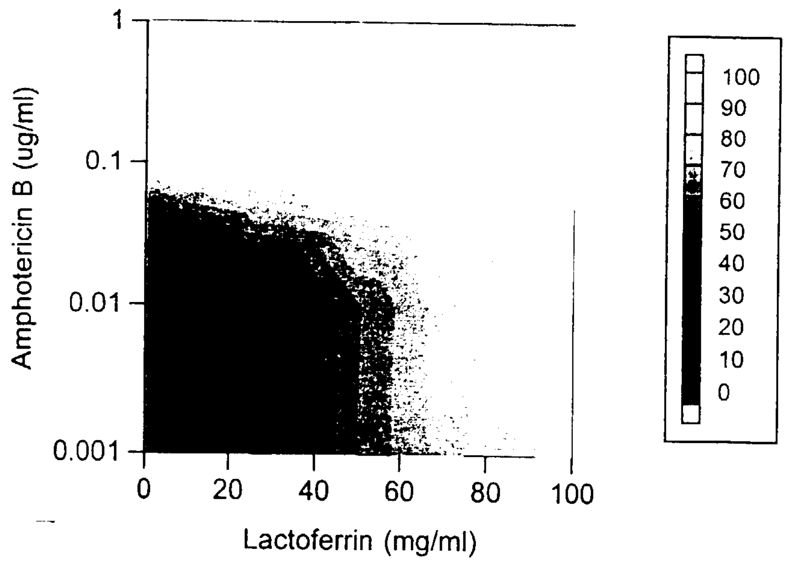

1
0.1
0.01
0.001
Amphotericin B (ug/ml)
0
20
40
60
80
100
Lactoferrin (mg/ml)
100
90
80
70
60
50
40
30
20
10
0


Fig. 12

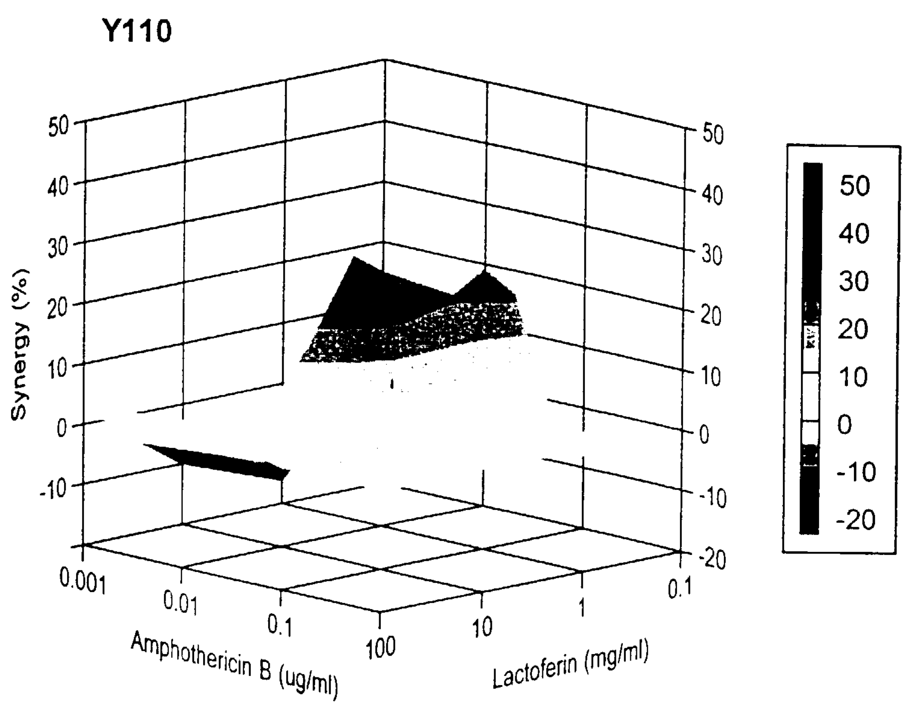

Y110
Synergy (%)
50
40
30
20
10
0
-10
-20
0.001
0.01
0.1
100
Amphothericin B (ug/ml)
10
1
0.1
Lactoferin (mg/ml)


Fig. 13

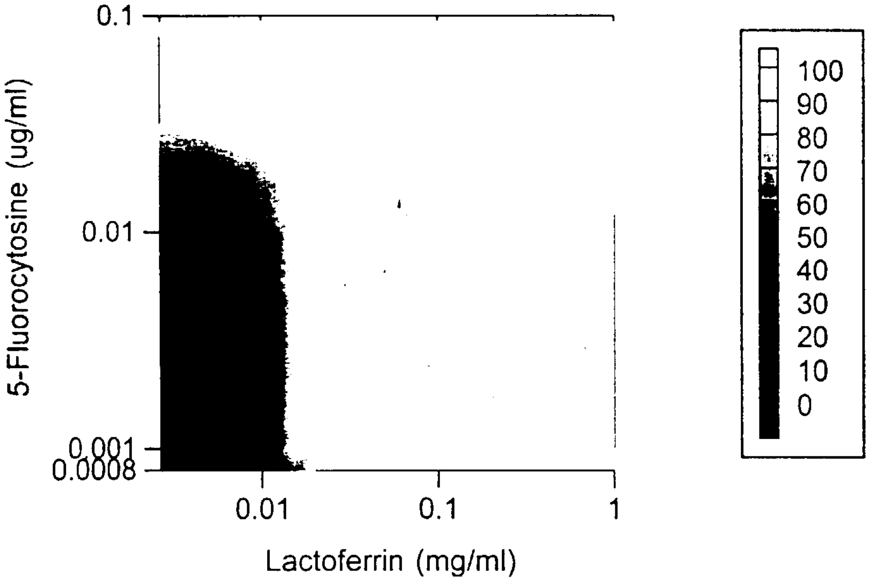

0.1
0.01
0.001
0.0008
5-Fluorocytosine (ug/ml)
0.01
0.1
1
Lactoferrin (mg/ml)
100
90
80
70
60
50
40
30
20
10
0


Fig. 14

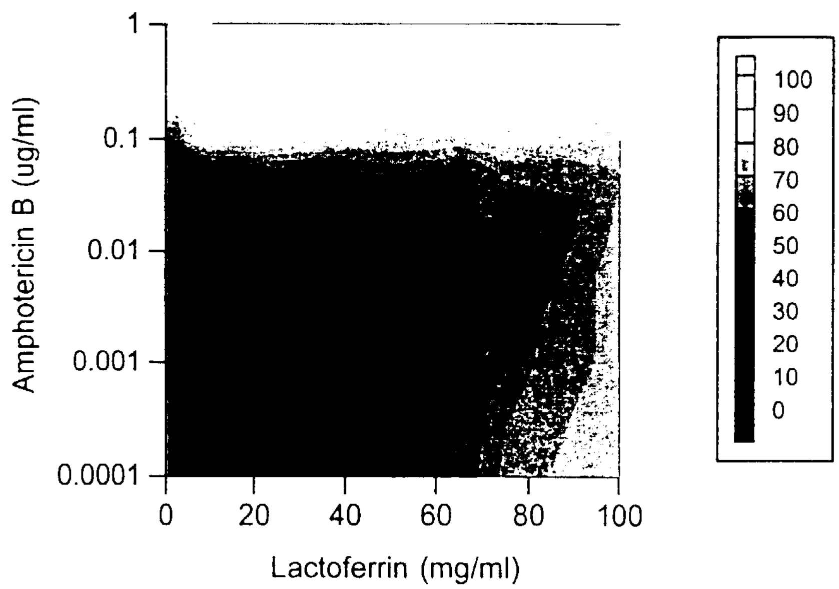

1
0.1
0.01
0.001
0.0001
Amphotericin B (ug/ml)
0
20
40
60
80
100
Lactoferrin (mg/ml)
100
90
80
70
60
50
40
30
20
10
0


Fig. 15

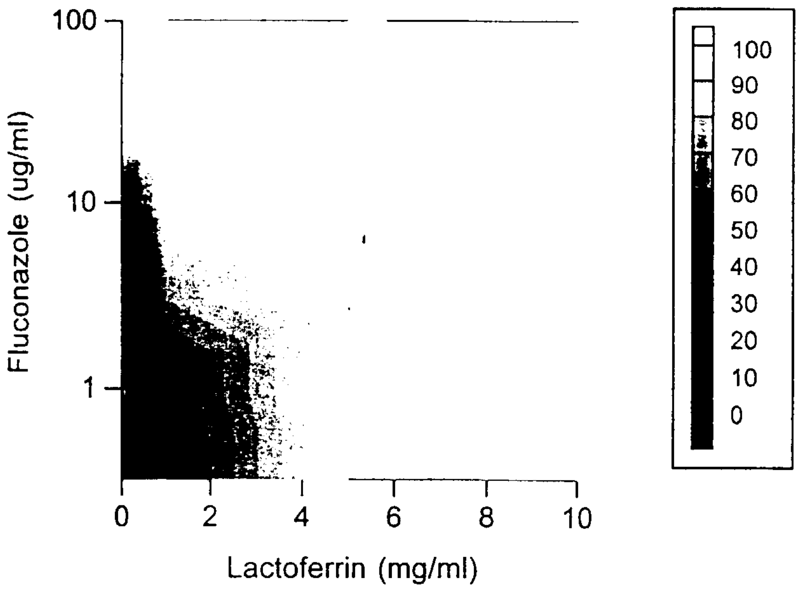
100
10
1
Fluconazole (ug/ml)
0
2
4
6
8
10
Lactoferrin (mg/ml)
100
90
80
70
60
50
40
30
20
10
0

Fig. 16

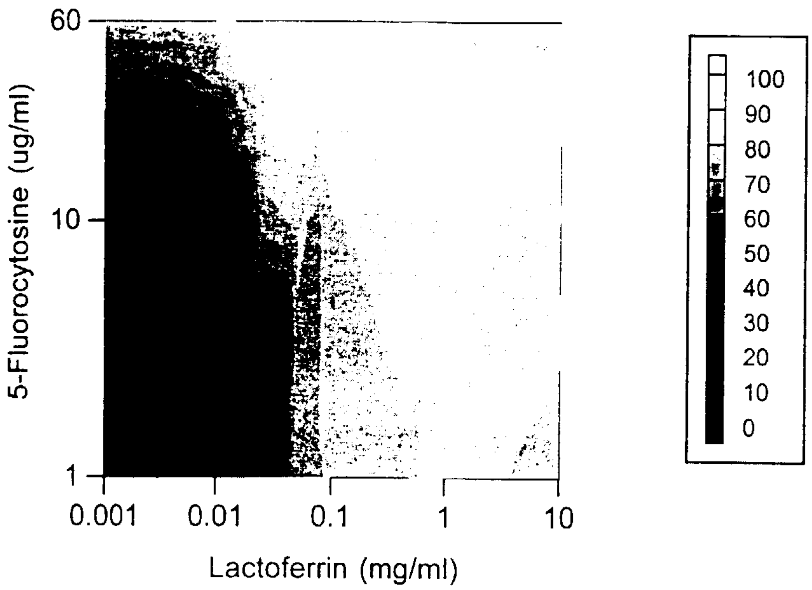
60
10
1
5-Fluorocytosine (ug/ml)
0.001
0.01
0.1
1
10
Lactoferrin (mg/ml)
100
90
80
70
60
50
40
30
20
10
0

Fig. 17

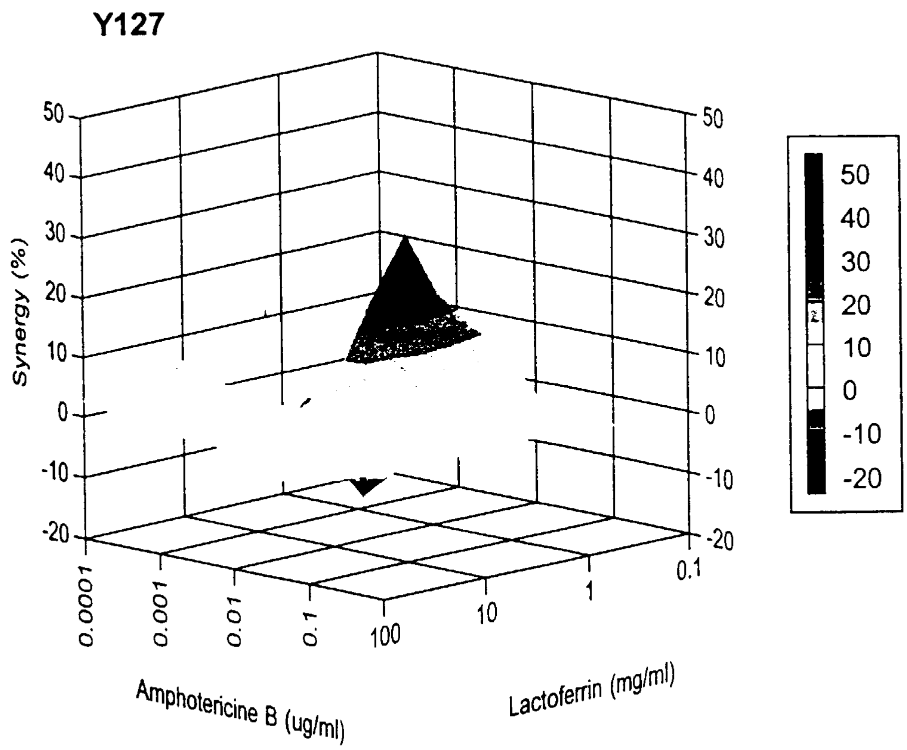
Y127
Synergy (%)
50
40
30
20
10
0
-10
-20
0.0001
0.001
0.01
0.1
100
10
1
0.1
Amphotericine B (ug/ml)
Lactoferrin (mg/ml)

Fig. 18

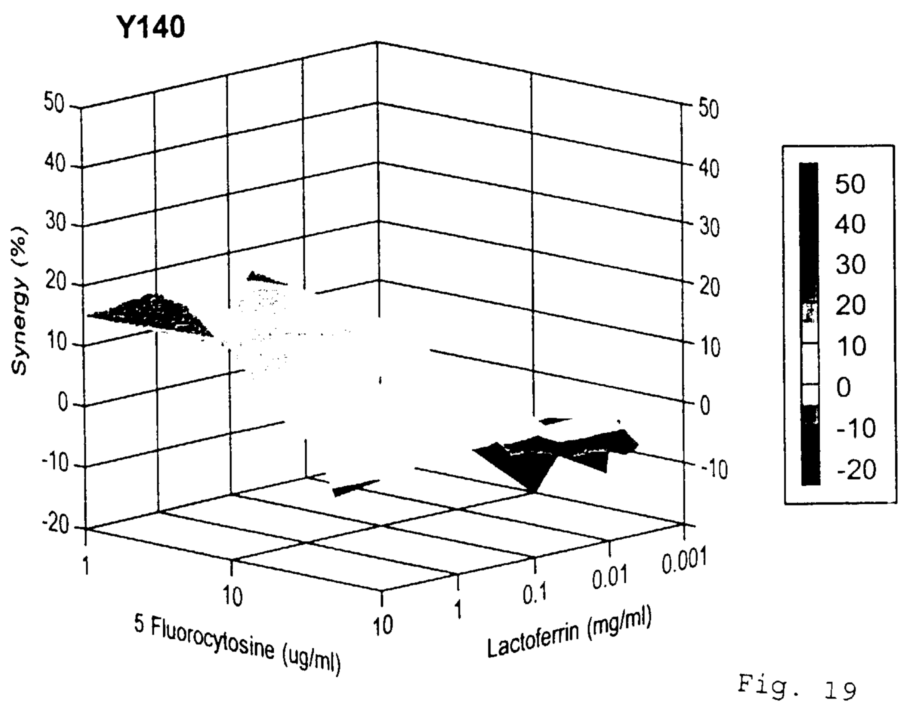
Y140
Synergy (%)
50
40
30
20
10
0
-10
-20
1
10
10
1
0.1
0.01
0.001
5 Fluorocytosine (ug/ml)
Lactoferrin (mg/ml)

Fig. 19

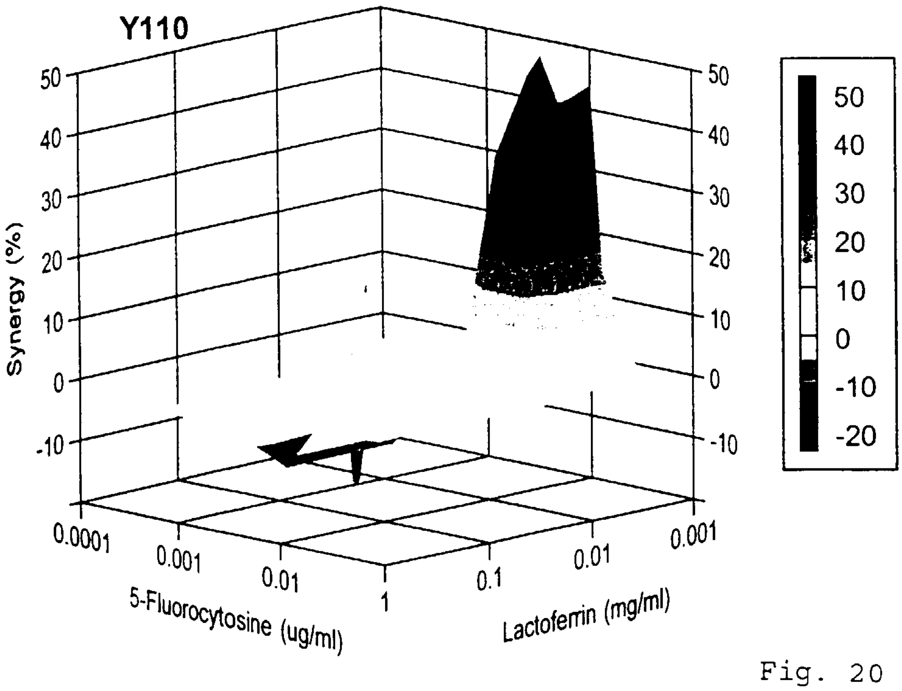
Y110
Synergy (%)
50
40
30
20
10
0
-10
-20
0.0001
0.001
0.01
1
5-Fluorocytosine (ug/ml)
0.1
0.01
0.001
Lactoferrin (mg/ml)

Fig. 20

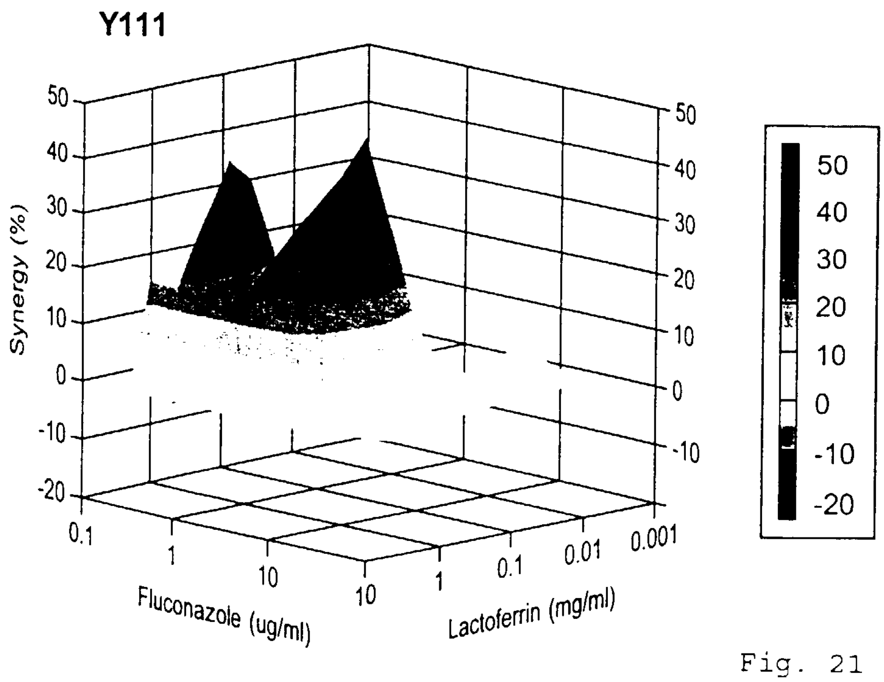
Y111
Synergy (%)
50
40
30
20
10
0
-10
-20
0.1
1
10
10
Fluconazole (ug/ml)
1
0.1
0.01
0.001
Lactoferrin (mg/ml)

Fig. 21

**Annex to the description**

SEQUENCE LISTING

(1) GENERAL INFORMATION:

(i) APPLICANT:
(A) NAME: N.V. Nutricia
(B) STREET: Eerste Stationsstraat 186
(C) CITY: Zoetermeer
(E) COUNTRY: The Netherlands
(F) POSTAL CODE (ZIP): 2712 HM

(ii) TITLE OF INVENTION: Pharmaceutical preparation for use in combatting or preventing surface infections caused by microorganisms

(iii) NUMBER OF SEQUENCES: 28

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 1:

```
Arg Trp Gln Trp Arg
1               5
```

(2) INFORMATION FOR SEQ ID NO: 2:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 2:

Arg Arg Gln Trp Arg
1 5

(2) INFORMATION FOR SEQ ID NO: 3:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 3:

Lys Val Ser Trp Arg
1 5

(2) INFORMATION FOR SEQ ID NO: 4:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 4:

Arg Asn Met Arg Lys
1 5

(2) INFORMATION FOR SEQ ID NO: 5:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 5:

Arg Trp Gln Glu Lys
1 5

(2) INFORMATION FOR SEQ ID NO: 6:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 6:

Arg Arg Trp Gln Trp Arg
1 5

(2) INFORMATION FOR SEQ ID NO: 7:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 7:

Arg Arg Arg Gln Trp Arg
1 5

(2) INFORMATION FOR SEQ ID NO: 8:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 8:

Lys Thr Val Ser Trp Arg
1 5

(2) INFORMATION FOR SEQ ID NO: 9:

(i) SEQUENCE CHARACTERISTICS:
(A) LENGTH: 6 amino acids
(B) TYPE: amino acid
(C) STRANDEDNESS:

(D) TOPOLOGY: linear

(ii) MOLECULE TYPE: peptide

(xi) SEQUENCE DESCRIPTION: SEQ ID NO: 9:

```
Lys Arg Asn Met Arg Lys
1               5
```

(2) INFORMATION FOR SEQ ID NO: 10:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 10:

```
Arg Trp Gln Glu Met Lys
1               5
```

(2) INFORMATION FOR SEQ ID NO: 11:

(i) SEQUENCE CHARACTERISTICS:
(A) LENGTH: 11 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 11:

```
Lys Thr Arg Arg Trp Gln Trp Arg Met Lys Lys
1               5                   10
```

(2) INFORMATION FOR SEQ ID NO: 12:

(i) SEQUENCE CHARACTERISTICS:
(A) LENGTH: 11 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 12:

```
Lys Ser Arg Arg Arg Gln Trp Arg Met Lys Lys
1               5                   10
```

(2) INFORMATION FOR SEQ ID NO: 13:

(i) SEQUENCE CHARACTERISTICS:
(A) LENGTH: 11 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 13:

```
Lys Thr Val Ser Trp Gln Thr Tyr Met Lys Lys
1               5                   10
```

(2) INFORMATION FOR SEQ ID NO: 14:

(i) SEQUENCE CHARACTERISTICS:
(A) LENGTH: 11 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 14:

```
Lys Thr Phe Gln Trp Gln Arg Asn Met Arg Lys
1               5                   10
```

(2) INFORMATION FOR SEQ ID NO: 15:

(i) SEQUENCE CHARACTERISTICS:
(A) LENGTH: 11 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 15:

Lys Thr Leu Arg Trp Gln Asn Arg Met Arg Lys
1 5 10

(2) INFORMATION FOR SEQ ID NO: 16:

(i) SEQUENCE CHARACTERISTICS:
(A) LENGTH: 20 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 16:

Lys Cys Arg Arg Trp Gln Trp Arg Met Lys Lys Leu Gly Ala Pro Ser
1 5 10 15

Ile Thr Cys Val
20

(2) INFORMATION FOR SEQ ID NO: 17:

(i) SEQUENCE CHARACTERISTICS:
(A) LENGTH: 20 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 17:

Lys Cys Arg Arg Trp Gln Trp Arg Met Lys Lys Leu Gly Ala Pro Ser
1 5 10 15

Ile Thr Cys Val
20

(2) INFORMATION FOR SEQ ID NO: 18:

(i) SEQUENCE CHARACTERISTICS:
(A) LENGTH: 20 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 18:

Lys Cys Phe Gln Trp Gln Arg Asn Met Arg Lys Val Arg Gly
Pro Pro
1 5 10
15

Val Ser Cys Ile
20

(2) INFORMATION FOR SEQ ID NO: 19:

(i) SEQUENCE CHARACTERISTICS:
(A) LENGTH: 19 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 19:

Lys Cys Phe Gln Trp Gln Arg Asn Met Arg Lys Val Gly Pro
Pro Val
1 5 10
15

Ser Cys Ile

(2) INFORMATION FOR SEQ ID NO: 20:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 20:

Phe Gln Trp Gln Arg Asn
1 5

(2) INFORMATION FOR SEQ ID NO: 21:

(i) SEQUENCE CHARACTERISTICS:
(A) LENGTH: 5 amino acids

(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 21:

Phe Gln Trp Gln Arg
1               5

(2) INFORMATION FOR SEQ ID NO: 22:

(i) SEQUENCE CHARACTERISTICS:
(A) LENGTH: 4 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 22:

Gln Trp Gln Arg
1

(2) INFORMATION FOR SEQ ID NO: 23:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 23:

Arg Arg Trp Gln Trp
1               5

(2) INFORMATION FOR SEQ ID NO: 24:

(i) SEQUENCE CHARACTERISTICS:
(A) LENGTH: 4 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 24:

Arg Arg Trp Gln
1

(2) INFORMATION FOR SEQ ID NO: 25:

(i) SEQUENCE CHARACTERISTICS:
(A) LENGTH: 4 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 25:

Trp Gln Trp Arg
1

(2) INFORMATION FOR SEQ ID NO: 26:

(i) SEQUENCE CHARACTERISTICS:
(A) LENGTH: 6 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 26:

Leu Arg Trp Gln Asn Asn
1 5

(2) INFORMATION FOR SEQ ID NO: 27:

(i) SEQUENCE CHARACTERISTICS:
(A) LENGTH: 5 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 27:

```
Leu Arg Trp Gln Asn
1               5
```

(2) INFORMATION FOR SEQ ID NO: 28:

(i) SEQUENCE CHARACTERISTICS:
(A) LENGTH: 4 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 28:

```
Leu Arg Trp Gln
1
```